(19) Europäisches Patentamt
European Patent Office
Office européen des brevets

(11) Publication number: 0 038 071 B1

(12) **EUROPEAN PATENT SPECIFICATION**

(45) Date of publication of patent specification: **14.11.84**

(21) Application number: **81102833.1**

(22) Date of filing: **13.04.81**

(51) Int. Cl.$^3$: **C 07 C 97/10,** C 07 C 131/00, C 07 C 103/52, C 07 C 102/04, G 01 N 33/56, C 07 B 23/00, A 61 K 47/00

(54) **New unlabelled and radioactive labelled derivatives of butylamino propiophenone, their preparation, immunogens obtained from the unlabelled derivatives and a process for immunogen preparation, antisera raised using the immunogens and a method for raising antisera, a radioimmunoassay method and a test kit for use in the assay method.**

(30) Priority: **14.04.80 US 140157**
**14.04.80 US 140159**
**15.04.80 US 140606**
**14.04.80 US 140158**
**14.04.80 US 140160**
**14.04.80 US 140165**

(43) Date of publication of application:
**21.10.81 Bulletin 81/42**

(45) Publication of the grant of the patent:
**14.11.84 Bulletin 84/46**

(84) Designated Contracting States:
**CH DE FR GB LI NL SE**

(56) References cited:
**DE-A-2 059 618**
**GB-A-1 011 289**
**GB-A-1 142 782**

(73) Proprietor: **THE WELLCOME FOUNDATION LIMITED**
**183-193 Euston Road**
**London NW1 2BP (GB)**

(72) Inventor: **Welch, Richard Martin**
**Katherine Court Springdale Estates, Rt. 6**
**Raleigh North Carolina 27609 (US)**
Inventor: **Butz, Robert Frederick**
**3619 Barcelona Avenue**
**Durham North Carolina 27707 (US)**
Inventor: **Scharver, Jeffrey Douglas**
**5202 Autumn Drive**
**Durham North Carolina 27712 (US)**
Inventor: **Phillips, Arthur Page**
**1400 Kershaw Drive**
**Raleigh North Carolina 27609 (US)**
Inventor: **Mehta, Nariman Bomanshaw**
**4207 Union Street**
**Raleigh North Carolina 27609 (US)**
Inventor: **Findlay, John William Addison**
**Rt. 2 Box 514 Cascade Drive**
**Chapel Hill North Carolina 27514 (US)**

(74) Representative: **Berg, Wilhelm, Dr. et al**
**Patentanwälte Dr. Berg Dipl.-Ing. Stapf Dipl.-Ing. Schwabe Dr. Dr. Sandmair Postfach 86 02 45**
**Stuntzstrasse 16**
**D-8000 München 86 (DE)**

**EP 0 038 071 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to new unlabelled and radioactive labelled derivatives of butylamino propiophenone, their preparation, immunogens obtained from the unlabelled derivatives and a process for immunogen preparation, antisera raised using the immunogens and a method for raising antisera, a radioimmunoassay method and a test kit for use in the assay method.

The present invention is primarily directed to a radioimmunoassay for clinical or experimental testing for the presence of a quantitation of bupropion [(±)-2-tert-butylamino-3′-chloropropiophenone], a pharmacologically active antidepressant compound, in biological fluids, including especially human sera or plasma.

The radioimmunoassay technique is finding increasing application for quantitation of drugs in biological fluids. Monitoring of plasma concentrations of drugs enables more precise dose administration to ensure efficacy. In the hospital laboratory setting, specific radioimmunoassay methods can offer considerable advantages such as improved sensitivity and specificity and, particularly, greater speed and sample capacity, over the generally more laborious methods of gas chromatography or thin-layer chromatography.

Accordingly, it is a purpose of the present invention to provide a radioimmunoassay procedure to determine the presence and concentration of bupropion in biological fluids, especially human sera or plasma.

It is another purpose of the invention to provide suitable radiolabelled agents for use in the radioimmunoassay.

It is another purpose of the invention to provide antibodies directed against bupropion also referred to as bupropion-specific antisera and as anti-bupropion sera. In this regard, it is a further purpose of the present invention to provide suitable immunogens for use to raise bupropion-specific antisera, and to provide methods suitable for raising such antisera.

The radioimmunoassay of the present invention is based on competition between bupropion and a fixed quantity of a distinguishable competitor of bupropion for a limited number of binding sites on bupropion-specific antibodies. The distinguishable competitor is preferably a labeled compound, such as a compound labeled with a radioactive isotope ("radiolabeled").

The drug and its radiolabeled competitor are added to the antibodies and the reaction mixture is allowed to equilibrate. When the antibody-bound portion is separated from the free drug and free radiolabeled competitor, then the amount of radioactivity present in the antibody-bound will be inversely related to the quantity of unlabeled drug originally added to the reaction mixture. Thus, the greater the amount of unlabeled drug added, the less radiolabeled competitor will be bound to the antibodies. Conversely, the amount of radioactivity present in the reaction mixture from which the antibody-bound portion has been removed will be directly related to the quantity of unlabeled drug originally added to the reaction mixture; i.e. the greater the amount of unlabeled drug added, the more radiolabeled competitor will remain in the reaction mixture from which the antibody-bound portion has been removed.

A standard curve can be produced by employing a range of known concentrations of unlabeled drug in a series of reaction mixtures while holding constant the amount of antibody and radiolabeled competitor employed. The standard curve allows the concentration of drug in an unknown sample to be interpolated from the amount of radioactivity present in the antibody-bound portion of the equilibrated reaction mixture to which it was added.

Thus, it can be seen that the radioimmunoassay of the present invention requires two principal reagents other than the unknown sample of biological fluid to be assayed. These are the radiolabeled competitor of the drug to be assayed, bupropion, and a bupropion-specific antiserum.

Bupropion-specific antisera

Antisera to be used in the radioimmunoassay of the present invention must have high specificity for bupropion, and poor recognition of (i.e., low cross-reaction with) other species which might be present in the biological fluid to be tested, e.g., metabolites of bupropion. According to one aspect of the present invention, suitable immunogens are described, which may be utilized to induce formation of antibodies specific to bupropion and having low recognition of other species in the medium.

Immunogens

The immunogens of the present invention are utilized to induce formation of antibodies specific to bupropion. The immunogens are presented, as by injection with suitable adjuvant, to the immune response system of a host animal. Improved titers can be obtained by a series of injections over a period of time. Suitable host animals include mammals such as horses, goats, guinea pigs and sheep. The preferred host animals are rabbits.

The immunogens of the present invention comprise suitable compounds linked *via* the aromatic ring or *via* the side chain ketone, to a suitable carrier material. Suitable carrier materials are known to those skilled in the art and include, for example, proteins; natural or synthetic polymeric materials such as polypeptides, e.g., polylsine and copolymers of amino acids; polysaccharides; and the like. Among

the suitable proteins useful in the practice of the present invention are mammalian serum proteins such as, for example, human gamma globulin, human serum albumin, rabbit serum albumin, and bovine gamma globulin. The preferred carrier material is bovine serum albumin (BSA).

While not intending to be bound by theory, it is presently believed that metabolites of bupropion in man involve reduction of the side-chain ketone with or without hydroxylation of the *t*-butyl group on the nitrogen. Accordingly, the present invention provides methods of making novel bupropion variants which can be linked to a suitable carrier material, preferably via the aromatic ring, thus leaving the side chain available for recognition by the host animal immune system.

According to another aspect of the present invention discussed below novel methods are provided for making the drug/carrier conjugate.

The new compounds provided by the present invention are of the formula I:

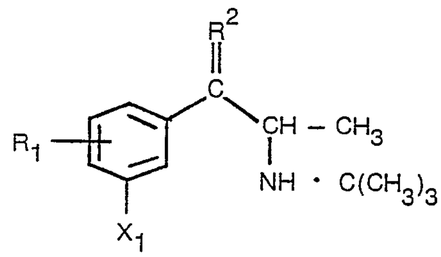

I

wherein

(1) $X_1$ is Cl, $R^2$ is oxygen and
$R^1$ is $-(CH_2)_n-OH$
or $-(CH_2)n-O-(CO)_m(CH_2)_pCOZ$
or $-O-(CH_2)_pCOZ$
or $-O-(CH_2)_pCOOR_{11}$
or is a radioisotope or

(2) $X_1$ is Cl, $R^1$ is H and
$R^2$ is $=N \cdot O(CH_2)_qCOZ$ or

(3) $X_1$ is a radioisotope, $R_2$ is oxygen and $R_1$ is OH in the 4-position

and wherein n is an integer of 1—5, m is 0 or 1, p is an integer of 1—4, q is an integer of 1—3, $R_{11}$ is a $C_1$—$C_3$ alkyl group and Z is OH or a group of formula:

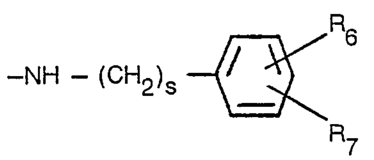

or

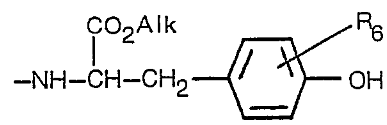

where $R_6$ is a radioactive nuclide, $R_7$ is OH, Alk is alkyl of 1 to 4 carbon atoms and s is an integer of 1—4.

The compounds of the present invention for use in immunogen preparation include compounds of Formula IA:

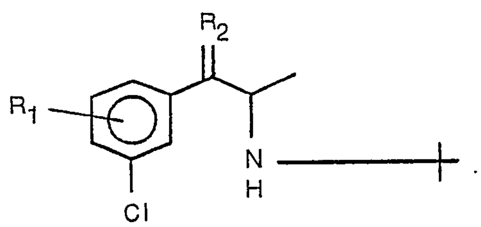

IA

wherein either $R_2$ is oxygen and $R_1$ is $(CH_2)_n-O-(CO)_m-(CH_2)_pCOOH$ where n is an integer from 0 to 5, m is 0 or 1, and p is an integer from 1 to 4; or $R_1$ is hydrogen and $R_2$ is $N-O-(CH)_q-COOH$ where q is an integer from 1 to 3. Included are each enantiomer and any mixture thereof. Preferred compounds include:

α-*t*-butylamino-3-chloropropiophenonecarboxymethyloxime, (compound 1);

α-*t*-butylamino-3-chloro-4-carbomethylpropiophenone, (compound 2); and

α-*t*-butylamino-3-chloro-4-γ-hydroxypropylpropiophenone hemisuccinate, (compound 3);

each of which has been found to be highly suitable for preparation of immunogens according to the present invention for the purpose of bupropion specific antisera preparation.

The preferred compound α-*t*-butylamino-3-chloropropiophenonecarboxymethyloxime, compound 1,

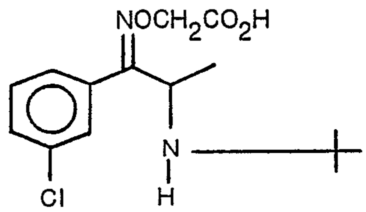

(and suitable analogs and derivatives as would be obvious in view thereof) can be prepared by reaction of bupropion with carboxymethoxylamine hemihydrochloride in the presence of anhydrous sodium acetate in suitable solvent, such as ethanol/water. Suitable varitions in the method and alternate reagents will be obvious to one skilled in the art in view of the instant disclosure.

The preferred compound *α-t*-butylamino-3-chloro-4-carboxymethoxypropiophenone, compound 2,

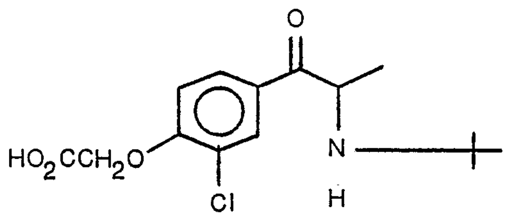

(and suitable analogs and derivatives as would be obvious in view thereof) can be prepared according to known methods by basic hydrolysis of *α-t*-butylamino-3-chloro-4-carboxymethoxypropiophenone methyl ester hydrochloride, compound 4, (or suitable corresponding intermediate) such as by reaction with potassium hydroxide.

Intermediate compound 4 can be prepared from the readily available starting materials *o*-chlorophenol and propionyl chloride which react with heating to give *o*-chlorophenyl propionate. Treatment of the product with aluminum chloride produces mixed reaction products including 3-chloro-4-hydroxypropiophenone. To a mixture of 3-chloro-4-hydroxypropiophenone and sodium methoxide disclosed in suitable solvent is added ethyl bromoacetate and reaction at reflux gives ethyl 2-(2-chloro-4-propionylphenoxy) acetate. Subsequent bromination in methanol- gives methyl 2-[2-chloro-4-(2-bromopropionyl)acetate which reacts with *t*-butylamine to give compound 4. Modifications of the method of synthesis such as selection of suitable and alternate solvents, reaction conditions and reagents, is well within the skill of the art in view of the present disclosure and such modifications do not bring the disclosed method outside the scope of the present invention.

Similarly in view of the present disclosure, it would be within the skill of the art to modify the above method to produce other intermediates for compounds of Formula I. To the extent there is a preferred embodiment of one or more aspects of the method of making compound 4, each is incorporated into the method of Example I.

An alternate method of synthesis of compound 4 is diagramatically illustrated in Synthesis Method A.

The preferred compound *α-t*-butylamino-3-chloro-4-*γ*-hydroxypropylpropiophenone hemisuccinate, compound 3,

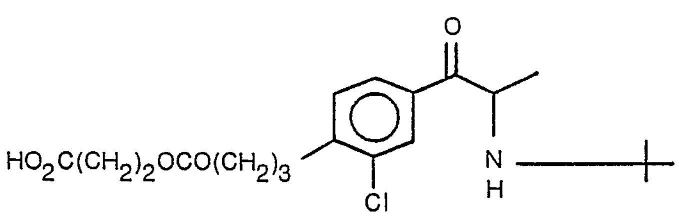

(and suitable analogs and derivatives as would be obvious in view thereof) is prepared by reaction of *α*-*t*-butylamino-3-chloro-4-(3-hydroxypropyl)propiophenone hydrochloride three-fourths hydrate, compound 5,

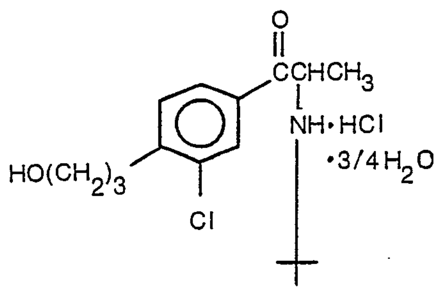 5

with succinic anhydride in pyridine to form the *o*-hemisuccinate. Intermediate compound 5 is suitably prepared from readily available starting materials. Accordingly, 3-chloro-4-methyl-benzonitrile, N-bromosuccinimide and benzoyl peroxide react at reflux with illumination to give 2-chloro-4-cyanobenzyl bromide. The product compound can be reacted with diethyl malonate in a solution of sodium in ethanol to give diethyl 2-(2-chloro-4-cyanobenzyl)malonate which, upon subsequent treatment with sodium chloride and water in DMSO produces ethyl 3-(2-chloro-4-cyanophenyl)propanoate. Treatment with suitable base, such as potassium hydroxide and alcoholic solvent gives the corresponding propanoic acid, 3-(2-chloro-4-cyanophenyl)propanoic acid which is then reduced to the corresponding alcohol, such as by treatment with $B_2H_6$ in tetrahydrofuran (THF). A mixture of the alcohol, 3-(2-chloro-4-cyanophenyl)propanol and ethyl magnesium bromide in dry ethyl ether is reacted at reflux to give 3-chloro-4-(3-hydroxypropyl)propiophenone which yields compound 5 upon bromination to $\alpha$-bromo-3-chloro-4-(3-hydroxypropyl)propiophenone and subsequent reaction of the bromoketone with an excess of t-butylamine in suitable solvent, such as $CH_3CN$.

In view of the present disclosure, it would be within the skill of the art to modify the above method to produce other intermediates for compounds of Formula I and to modify it by selection of alternate suitable solvents, reagents and reaction conditions, none of which modifications take it outside the scope of the present invention. To the extent there is a preferred embodiment of some aspects of this method, each is incorporated into the method of Example II.

The application of the above described method of making compound 3, to other compounds within formula I having the formula

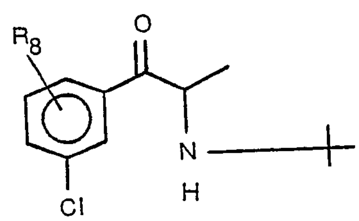

wherein $R_8$ is $(CH_2)_n—O—CO(CH_2)_p—CO_2H$ where n and p are as defined for formula I, would be within the skill of the art. Thus such compounds can be made by reacting an intermediate compound of formula

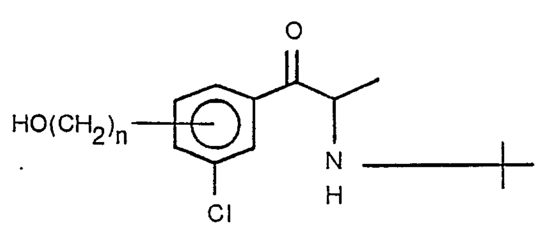

wherein n is as defined for formula I, with

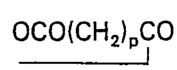

wherein p is as defined for formula I.

The above described method of making compound 2 is readily applied to make other compounds within formula I having the formula

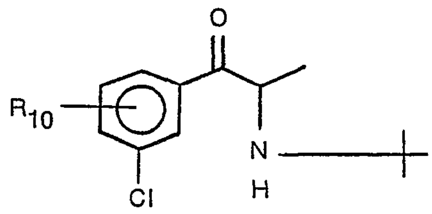

wherein $R_{10}$ is $O—(CH_2)_pCOOH$ where p is as defined above. Accordingly, such compounds can be made by basic hydrolysis of an intermediate compound of formula

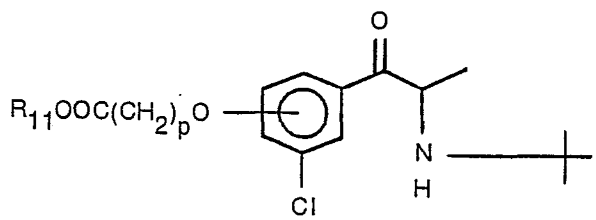

where p is as defined above and $R_{11}$ is alkyl of 1 to 3 carbons. Preferably potassium or sodium hydroxide is used.

Immunogen Preparation

The coupling of a bupropion variant of the present invention to the immunogenic carrier material can be readily accomplished utilizing techniques well known to the skilled in the art. Thus, for example, one suitable technique comprises dissolving the bupropion variant, the carrier material and a suitable coupling agent in suitable inert solvent to react. As discussed above, for assay of human sera or plasma the bupropion variant should be coupled to the carrier material in such a way that regions believed to be affected by metabolic changes in man are presented for ready recognition by the host animal immune system. Thus, coupling is preferably *via* the aromatic ring. Accordingly, as one aspect of the present invention, there are now disclosed preferred routes to conjugates of the bupropion variants of the present invention such as compounds 2 and 3 disclosed above, linked *via* the aromatic ring to suitable carrier material. Such conjugates present the bupropion side chain for recognition by the host animal immune system.

A first preferred conjugate comprises compound 2 coupled to BSA using a suitable, water-soluble carbodiimide catalyst. Present understanding suggests that the carbodiimide catalyzes the formation of peptide bonds between the free acid moiety of compound 2 and $\varepsilon$-amino group of lysyl residues of BSA. A preferred carbodiimide is 1-ethyl-3-(3-dimethylaminopropyl)-carbodiimide hydrochloride ("EDC"). In view of the present disclosure, it is within the skill of the art to modify this method of forming the conjugate to apply it to other compounds within formula I or to employ alternate suitable catalyst, none of which modifications take the method outside the scope of the present invention. To the extent there is a preferred embodiment of some aspect of this method, it is incorporated into the method illustrated in Example III.

A second preferred conjugate comprises compound 3 coupled to BSA using the mixed anhydride method. Thus, for example, compound 3 can be reacted with triethylamine and isobutylchloroformate in dimethylformamide to form the mixed anhydride. Upon addition to BSA in sodium carbonate the desired conjugate is formed. This method is illustrated in Example IV and in view of the present disclosure it is within the skill of the art to apply the method of Example IV to other compounds of formula I. Modifications such as, for example, in the choice of reaction conditions and reagents, will be obvious to the skilled in the art and are not outside the scope of the present invention. To the extent there is a preferred embodiment of some aspects of this method, it is incorporated into the method illustrated in Example IV.

The possibility is seen that ring-hydroxylated metabolites of bupropion could exist in the biological fluid to be assayed. The antisera raised utilizing the immediately above described conjugates might to some extent cross react with such metabolities. While the efficacy of the radioimmunoassay of the present invention would be maintained notwithstanding such possible cross-reaction, there is also provided, as one aspect of the present invention, the preparation of conjugates comprising bupropion variant linked to suitable carrier material, preferably BSA, via the side chain ketone. Utilizing such immunogen provides antisera which would not cross-react substantially with ring-hydroxylated metabolites of bupropion. Such antisera could be expected to cross-react with side-chain alcohol metabolities.

Accordingly, a third preferred conjugate comprises compound 1 coupled to BSA employing a water soluble carbodiimide method. This method is illustrated in Example V.

The radioimmunoassay of the present invention is based on the finding that bupropion will successfully compete with radiolabeled bupropion, or a suitable radiolabeled competitor of bupropion according to the present invention, for binding to antisera raised by presenting a suitable bupropion variant-carrier material conjugate to the immune system of host animals, such as rabbits.

Radiolabelled Bupropion Competitor

Radioactively labeled bupropion or the radioactivity labeled bupropion competitors of the present invention can be labeled in the manner well known to the art with any suitable radionuclide. A listing of the radionuclides which are now conventionally in use in reagents and which may be used in this invention are listed in the index of radionuclides found on page 81 of the 1978 edition of the Catalogue of the New England Nuclear Corporation, Boston, Massachusetts, U.S.A.. (New England Nuclear, 1977) incorporated herein by reference. Among radionuclides which are preferred in this invention the following may be mentioned: hydrogen-3- (tritium) and the radio isotopes of iodine ($^{123}I$, $^{124}I$, $^{125}I$, $^{126}I$, $^{128}I$, $^{130}I$, $^{131}I$ and $^{132}I$) with $^{125}I$ and $^{3}H$ being preferred from considerations of availability, half life and specific activity and/or the ability of these to be readily detected using a conventional gamma counter usually available in hospitals and sold by Packard Instruments or others.

The radiolabeled competitors of the present invention are of Formula II:

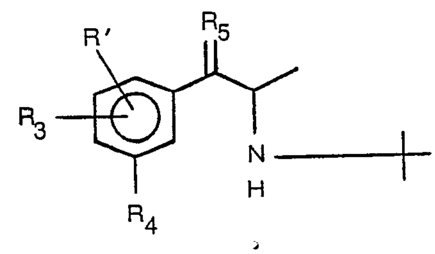

II

wherein R′ is a suitable radioisotope as described above and $R_3$ is hydrogen, $R_4$ is Cl, and $R_5$ is oxygen, or R′ is hydrogen and either a) $R_3$ is H and $R_4$ is Cl and $R_5$ is $=NO(CH_2)_2CO$—Ƶ where q is as defined for formula I and Ƶ is a group having formula III

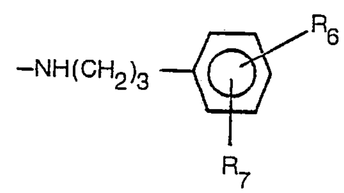

III

wherein $R_6$ is a suitable radioisotope as described above, $R_7$ is hydroxyl, and s is an integer from 1 to 4; or Ƶ is a group having formula III (a)

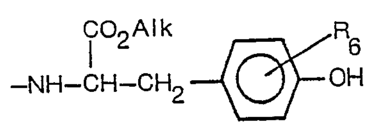

III (a)

wherein $R_6$ is a suitable radioisotope as described above, and Alk is a lower alkyl containing 1 to 4 carbons; b) $R_4$ is Cl, $R_5$ is oxygen, and $R_3$ is $(CH_2)_n$—O—$(CO)_m$—$(CH_2)_p$—CO—Ƶ where n, m, and p are as defined for formula I and Ƶ is as defined above; or c) $R_3$ is hydroxy, $R_5$ is oxygen and $R_4$ is a suitable radioisotope as defined above.

Most preferably, the radiolabeled competitor is selected from

*α-t*-Butylamino-3-chloropropiophenone-carboxymethyloxime-$^{125}I$-tyramine amide, (Compound 6)

*α-t*-Butylamino-3-chloro-4-carbomethoxypropiophenone-$^{125}I$-tyramine amide (compound 7)

*α-t*-Butylamino-3-chloro-4-*γ*-hydroxypyropylpropiophenone-hemisuccinate-$^{125}I$-tyramine amide (compound 8)

$^{125}I$-*α-t*-Butylamino-3-iodo-4-hydroxypropiophenone (compound 9)

*α-t*-Butylamino-3-chloro-2-[$^{3}H$]-propiophenone hydrochloride (compound 10)

In view of the present disclosure, the radiolabeled competitors of the present invention can be made according to methods well known to those skilled in the art. Thus, for the preparation of compound 6, compound 1 can be coupled to tyramine using dicyclohexylcarbodiimide to catalyze the formation of a peptide bond between the free acid of compound 1 and the primary amino group of tyramine. The product is subsequently iodinated in accordance with the Hunter-Greenwood

Chloramine-T Method, described in W. M. Hunter and F. C. Greenwood, Nature, *194*, 495 (1962) which is incorporated herein by reference in its entirety.

Preferred radiolabeled compound 7 is prepared from compound 2 and tyramine using the mixed anhydride method. Similarly, compound 8 can be prepared from compound 3 using the mixed anhydride method. In each case, the starting reagent is reacted with TEA and isobutyl chlorofomate in DMF to form the mixed anhydride. The reaction mixture is then added to tyramide and the product iodinated as described above.

The application of this method to other compounds of formula III would be within the skill of the art. Thus, compounds within formula III having the formula

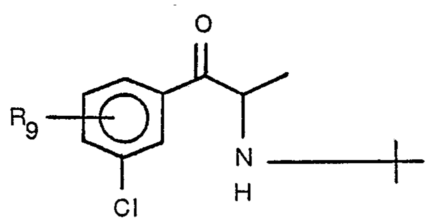

wherein $R_9$ is $(CH_{22})_n$—O—$CO(CH_2)_pCO$—X where n, p and X are as defined above, can be made by reacting a compound of formula 1 with trialkylamine and alkyl haloformate to form the mixed anhydride and subsequently adding the reaction mixture to $HOC_6H_4(CH_2)_sNH_2$ wherein s is as defined above. The product is subsequently iodinated, preferably with $^{125}I$. The trialkylamine preferably comprises an alkyl moiety of about 1 to 4 carbon and the alkyl moiety of alkyl haloformate preferably has 1 to 7 carbon. Triethylamine and isobutyl chloroformate are most preferred.

In this Specification TEA means triethylamine, DMF means dimethylformamide, DMSO means dimethylsulfoxide, BSA means bovine serum albumin.

Similarly, compounds within formula II having the formula

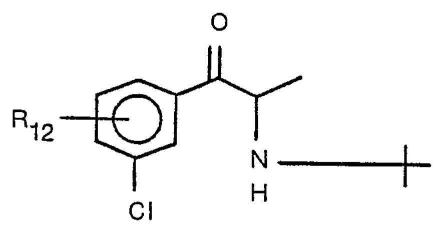

wherein $R_{12}$ is O—$(CH_2)_pCO$—Ƶ where p is as defined above and Ƶ is III or IIIa

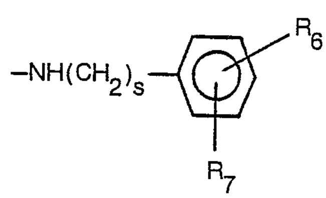

III

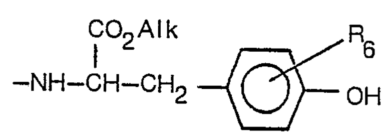

IIIa

where $R_6$, $R_7$, s and Alk are as defined above, can be made by reacting a compound of formula I with trialkylamine alkyl haloformate, as those terms were used above, to form the mixed anhydride. Subsequently adding the reaction mixture to $HOC_6H_4(CH_2)_sNH_2$ where s is as defined above or to $HOC_6H_4CH_2(CO_2Alk)NH_2$ where Alk is as defined above and then iodinating preferably with $^{125}I$ gives the desired radiolabeled competitor compound.

Compound 9 can be prepared by demethylation of $\alpha$-*t*-butylamino-4-methoxypropiophenone

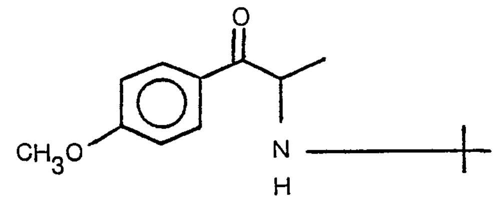

followed by iodination of the phenolic product.

Compound 10 is $^3H$ labeled bupropion and can be prepared as shown herein.

The Bupropion Radioimmunoassay Procedure

Preferably for high specificity, the antiserum is in each case raised to a bupropion variant which corresponds to the radiolabeled competitor used in the assay. Thus, radiolabeled competitor compound 6, is preferably used in conjunction with anti-($\alpha$-t-butylamino-3-chloropropiophenone-carboxymethyloxime-BSA) sera, ("anti-compound 1 sera"). Compound 7 and compound 8 are each preferably used with antisera raised to an immunogen wherein the bupropion variant is linked to the carrier material *via* the aromatic ring. Thus, each is preferably used with either anti-compound 2 sera or with anti-compound 3 sera. Radiolabeled competitor compounds 9 and 10 also are each used preferably with antisera raised to immunogens linked via the aromatic ring to the carrier material. Competition between the bupropion, if any, present in the biological fluid being assayed and the radiolabeled competitor proceeds in such a manner that equilibrium is achieved corresponding to the relative-concentration of bupropion and radiolabeled competitor in the assay mixture.

The present invention is also based in part upon the discovery that once the competition of bupropion and radiolabeled competitor has proceeded for the desired time, preferably to equilibrium, the antibody bound portion of the drug and competitor can be separated from the free portion of the drug and competitor. Following such separation, the amount of radioactivity in the antibody bound portion gives a measure of the amount of unlabeled bupropion which was present in the test mixture.

According to the preferred radioimmunoassay of the present invention, a standard curve is constructed by running the assay on two or more, preferably four to 8 solutions, each having a different known bupropion concentration within a suitable concentration range. In addition, an assay is run with only the radiolabeled competitor without the addition of bupropion, such that the maximum possible binding can be quantified. The assay for each standard solution can be expressed as a percent of maximum binding and plotted on a graph against the concentration of bupropion in the assayed sample. The value obtained upon assay of the unknown biological sample expressed as percent of maximum binding can then be used to interpolate its bupropion concentration from the standard curve.

Preferably, duplicate measures of standard bupropion solutions, preferably in blank plasma, are prepared such as by pipetting equal amounts of each standard solution into plastic tubes. In addition, duplicate "nonspecific binding tubes", that is tubes which will not receive antisera, and duplicate "maximum binding tubes", that is tubes which will not receive unlabelled bupropion, also each receive a like measure of blank plasma. While it is indicated that each sample is assayed in duplicate, this is merely preferred for greater accuracy and the average value for each pair is used. If desired, the assay may even be performed with triplicate samples. After being placed in an ice bath, all tubes receive a portion of radiolabeled competitor and subsequently all tubes, except non-specific binding tubes, receive anti-bupropion serum raised to an appropriate immunogen of the present invention. Preferably, the radiolabeled competitor and antiserum are in suitable assay buffer, such as phosphate buffered isotonic saline containing EDTA and gelatin. The non-specific binding tubes receive blank assay buffer.

Following incubation when the bupropion and the competing radiolabeled competitor in the assay mixture have substantially reached equilibrium, antibody-bound radiolabeled competitior is separated from the free portion thereof by any suitable means such as are known to the skilled in the art. In one preferred method a complex is formed between protein in the assay mixture and another added protein by incubation, for example, overnight at ambient temperature. The complex precipitates out and can be centrifuged to a pellet for measurement of radioactivity. Alternately, ammonium sulfate can be added to get a faster precipitation.

However, a novel and useful feature of the radioimmunoassay of the present invention is the ethanol precipitation of antibody-bound $^{125}I$-bupropion competitor from the equilibrium assay mixture. In this novel and preferred method for quick precipitation, the assay mixture must be kept ice-cold, such as by immersion in an ice bath. Absolute ethanol is added to the ice-cold mixture and quickly precipitates all the protein in the assay mixture. The precipitate can be pelletized and thus use of ethanol precipitation is significantly advantageous in allowing quantitation of $^{125}I$ in the protein pellets.

The quantitation of the antibody-bound radiolabeled competitior such as, for example, by beta or gamma radiation count of the antibody-bound $^3H$ or $^{125}I$ labeled competitor respectively minus the average count obtained for the non-specific binding tubes, is expressed as a percentage of the quantitation of the maximum binding tubes (also minus the average count obtained for the non-specific binding tubes). Using the results obtained for the tubes containing standard bupropion solution

samples, a standard curve can be constructed as already described. The assay procedure will be further explained by illustration in the examples.

The antisera raised to the above described ring functionalized immunogens have been found to provide excellent specificity. Cross reaction with the bupropion metabolites believed to occur in man, has been found to be of a low level.

The novel immunogens and antibodies of the present invention may be utilised in conjunction with conventional additives, buffers, stabilizers, diluents, or in combination with other physiologically active or inactive substances.

As one aspect of the present invention a kit is provided, such as for a mercantile unit, for practicing the radioimmunoassay of the present invention. Such kit comprises at least one container, such as, for example, a test tube, containing bupropion-specific antisera and bupropion competitor. In one embodiment, the antisera, presented, for example, in freeze dried form, is adhered to a first portion of the container and bupropion competitor is adhered to a first portion of the container and bupropion competitor is adhered to a second, separate portion of the container. In such embodiment, any suitable adhesive means can be used, such as, for example, a water soluble adherent which will not interfere with the bonding of bupropion or of the competitor to the antisera. Alternately, each reagent can be presented individually, each in one or more separate containers. The kit can also comprise, in the same or different container(s), standard amount(s) of bupropion, antisera and competitor. This would be preferred for example, where a standard curve is to be constructed.

## Drawings

In the accompanying drawings

Fig. 1 is a standard curve showing inhibition of the binding of radiolabelled competitor as a function of bupropion concentration and is explained in more detail in Example 12 below;

Fig. 2 shows standard curves obtained using certain $^{125}I$ iodinated bupropion competitors and antisera according to the present invention and is explained in more detail in Examples 13—17 below.

Whenever reference is made in this Specification to alkyl or a group comprising alkyl, the alkyl moiety thereof contains 1 to 6 carbon atoms unless otherwise specified and *t*-butylamino means *tert*-butylamino.

The following Examples are given to illustrate the invention.

## Example I

Preparation of α-*t*-butylamino-3-chloro-4-methoxycarbonylmethoxypropiophenone methyl ester hydrochloride, compound 4

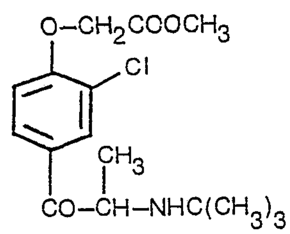

A. *O*-Chlorophenyl propionate

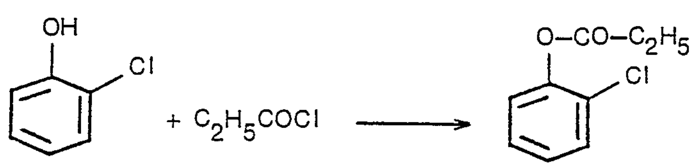


*o*-Chlorophenol (64 g, 0.5 mole) and propionyl chloride (50 g, 0.55 mole) were mixed at room temperature and then heated at 100°C (steam bath) for 2—3 hrs. Hydrogen chloride gas was evolved. After 2—3 hrs the reaction mixture was distilled *in vacuo* and gave 78.8 g (86%) of *o*-chlorophenyl propionate, bp 11 mm = 111°C.

Anal for $C_9H_9O_2Cl$ M.W. 184.63
Calcd: C, 58.54; H, 4.91
Found: C, 58.31; H, 4.88

B. 3-chloro-4 -hydroxpropiophenone

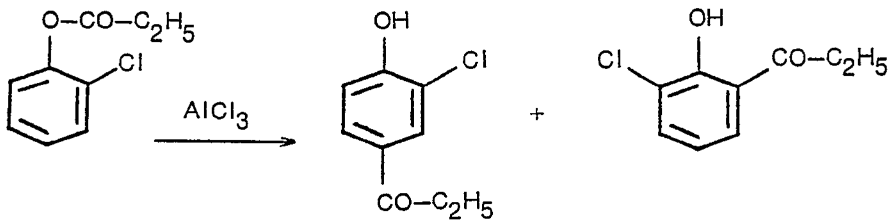


To anhydrous aluminium chloride (37 g, 0.27 mole), *o*-chlorophenyl propionate (24.8 g, 0.13 mole) was added rapidly (vigorous reaction). The reaction mixture was heated at 120—130°C (in a metal bath) for 30—45 min. After cooling, the aluminum chloride reaction mixture was decomposed with a mixture of ice and concentrated HCl. The solid organic material was filtered off by suction, and was washed with much cold water giving 18—20 g of solids. This product was recrystallized several times from ethyl acetate-hexane and (2) 5—6 g from evaporation of the ethyl acetate-hexane filtrates.

The 13—14 g of hexane insoluble material was recrystallized several times from mixtures of ethyl acetate and hexane. It was charcoaled while in the hot ethyl acetate solution and gave finally 3-chloro-4-hydroxypropiophenone (12 g, 48.4%) mp 114—115°C. (D. Chakravarti and B. Majurndar., *J. Indian Chem. Soc., 16,* 151—159 (1939) reported a mp of 80°C for this product)

Anal for $C_9H_9O_2Cl$ M.W. 184.63
Calcd: C, 58.54; H, 4.91
Found: C, 58.41; H, 4.76.

The material from the ethyl acetate-hexane soluble filtrate after several recrystallizations from hexane gave 2-hydroxy-3-chloropropiophenone (4.3 g, 17.3%) mp 42—45°C.

Anal for $C_9H_9O_2Cl$ M.W. 184.63
Calcd: C, 58.54; H, 4.91
Found: C, 58.42; H, 4.76

C. Ethyl 2-(2-chloro-4-propionylphenoxy) acetate

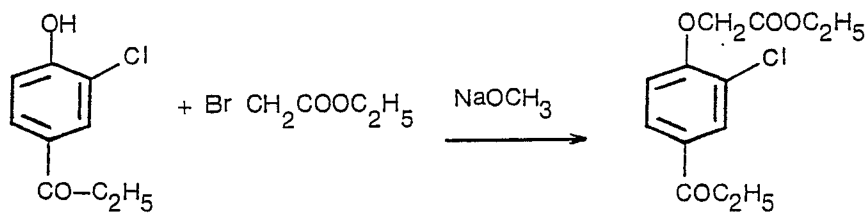


Sodium methoxide (2.5 g, 0.045 mole) was dissolved in absolute ethanol (40 ml) 3-Chloro-4-hydroxypropiophenone (7.4 g, 0.04 mole) was added and the mixture warmed for 1—2 minutes. Ethyl bromoacetate (7.3 g, 0.44 mole) was added, and the reaction mixture was refluxed at 100°C (steam bath) for 2—3 hrs. Most of the ethanol was evaporated, ice and water were added and the mixture was neutralized to pH 4—5 with dilute HCl. Cooling and scratching of the flask initiated crystallization. The resulting solid was dissolved in excess ether, washed 3x with 5% NaOH, then with water. The ethereal solution was dried over solid sulfate (anhydrous), filtered and evaporated and gave 9—10 g of crude material. Several recrystallizations from ethyl acetate gave ethyl 2-(2-chloro-4-propionylphenoxy)acetate (8.3 g, 77%), mp 129—130°C.

Anal for $C_{13}H_{15}O_4Cl$ M.W. 270.72
Calcd: C, 57.67; H, 5.59
Found: C, 57.67; H, 5.43.

D. Methyl 2-[2-chloro-4-(2-bromopropionyl)phenyl]acetate.

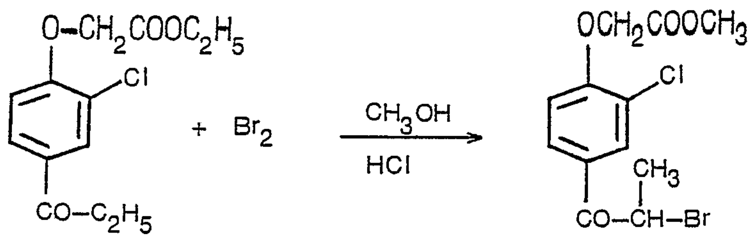


Ethyl-2-(2-chloro-4-propionylphenoxy)acetate (4 g) was dissolved in methanol (40—50 ml). HCl (2 drops, conc) was added, followed by the dropwise addition over 5—10 minutes of bromine (2.62 g, 0.016 mole). After the bromine addition was complete the mixture was heated on a steam bath for 15—30 minutes allowing much of the solvent to evaporate. The residue was washed with cold water and the remaining solid recrystallized several times from hexane to give methyl 2-[2-chloro-4-(2-bromopropionyl)phenoxy]acetate (4.6 g, 90%), mp 63—65°C.

Anal for $C_{12}H_{12}O_4Cl$ Br M.W. 335.69
Calcd: C, 42.93; H, 3.60
Found: C, 43.83; H, 3.46

E. Methyl 2-[2-chloro-4-(2-t-butylaminopropionyl)phenoxy] acetate hydrochloride

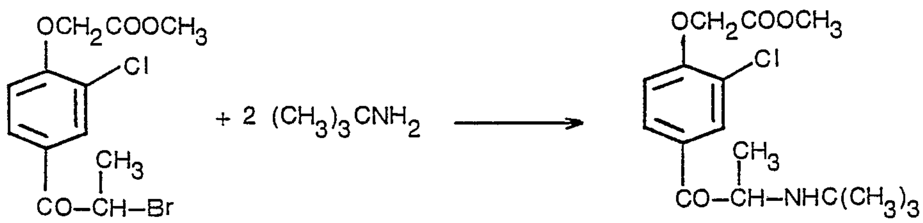

To methyl 2-[2-chloro-4-(2-bromopropionyl)phenoxy]acetate (3.3 g, 0.01 mole) in acetonitrile (20 ml) t-butylamine (3.3 g, 0.044 mole) was added, and the reaction mixture was left overnight at 40°C (near a warm steam bath). In the morning the mixture was heated on a steam bath, allowing solvent and excess amine to evaporate (to dryness). The residue was treated with cold water and NaOH to pH 11 and extracted quickly with ether. The ethereal solution was dried over anhydrous sodium sulfate, filtered, and evaporated to remove ether and any traces of t-butylamine. The residue was redissolved in ether and precipitated as the hydrochloride salt by the addition of a little alcoholic HCl. The white crystalline solid was recrystallized two times from mixtures of methanol-ethyl acetate-ether to give methyl 2-[2-chloro-4-(2-t-butylaminopropionyl)phenoxy]acetate hydrochloride (2.5 g, 68.5%), mp 213—215°C.

Anal for $C_{16}H_{23}NO_4Cl_2$ M.W. 364.29
Calcd: C, 52.76; H, 6.36; N, 3.85; 0.17.57; Cl, 19.47
Found: C, 52.48; H, 6.42; N, 3.71; Cl, 19.36.

Example II

Preparation of $\alpha$-*t*-butylamino-3-chloro-4-$\gamma$-hydroxypropylpropiophenone hemisuccinate, compound 3

A. 2-Chloro-4-cyanobenzyl bromide

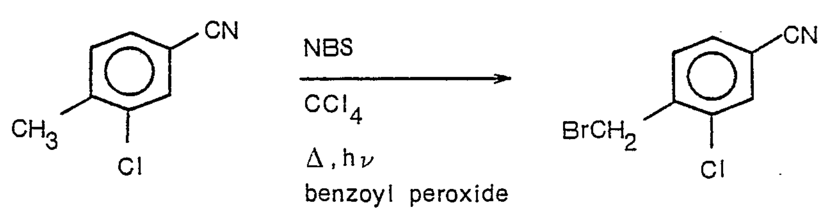


A mixture of 3-chloro-4-methyl-benzonitrile (30.0 g, 0.2 mol), N-bromosuccinimide (36.0 g, 0.21 mol) and benzoyl peroxide (100 mg) in 600 mL of $CCl_4$ was refluxed with illumination for 72 h. N-Bromosuccinimide (5.0 g) was added and the reaction was continued for an additional 24 h. The mixture was cooled, filtered and evaporated. Recrystallization from heptane gave pure 2-chloro-4-cyanobenzyl bromide: yield 24.5 g, mp 83—85°C. *Anal.* calcd for $C_8H_5$ BrClN; C, 41.69; H, 2.19; N, 6.07. Found: C, 41.48; H, 1.80; N, 5.76.

B. Diethyl 2-(2-chloro-4-cyanobenzyl)malonate.

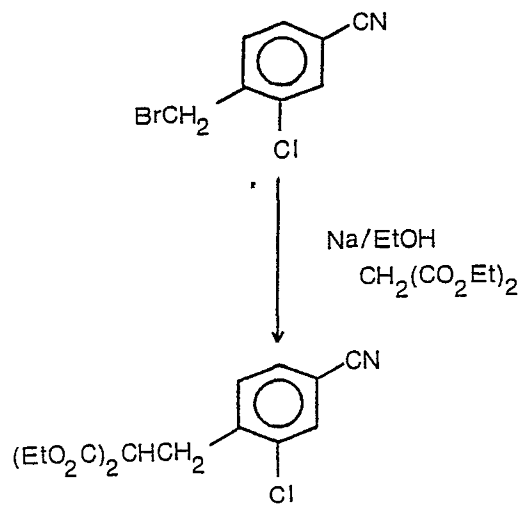


To sodium (2.0 g, 0.09 mol) dissolved in ethanol was added diethylmalonate (15.7 g, 0.1 mol) followed by 2-chloro-4-cyanobenzyl bromide (20.0 g, 0.09 mol). The mixture was refluxed for 3.5 h. The solvent was evaporated and 120 mL of water and 3.6 mL of concentrated hydrochloric acid was added to the residue. Separation and distillation gave diethyl 2-(2-chloro-4-cyanobenzyl)malonate as an oil; 12.5 g; bp 140—142°C (0.1 mm Hg).

Anal for $C_{15}H_{16}ClNO_4$
Calcd: C, 58.16; H, 5.21; N, 4.52
Found: C, 58.07; H, 5.22; N, 4.52.

C. Ethyl 3-(2-chloro-4-cyanophenyl)propanoate

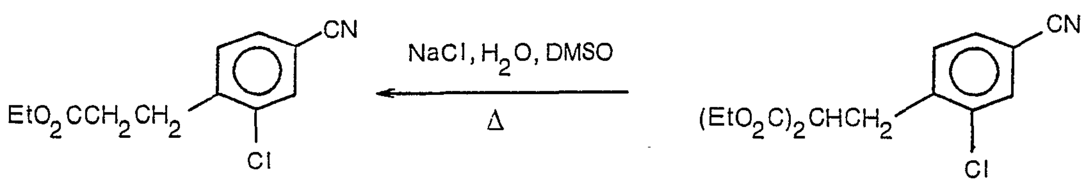


A mixture of dieethyl 2-(2-chloro-4-cyanobenzyl)malonate (9.0 g, 0.03 mol), sodium chloride (2.12 g, 0.04 mol), and water (0.86 g 0.05 mol) in 30 mL of DMSO was heated to 135°C and then the temperature was gradually raised to 170°C over a 3 h period. Evaporation and distillation gave ethyl 3-(2-chloro-4-cyanophenyl)propanoate as an oil: 5.8 g; bp 138—140°C (0.1 mm Hg)

Anal for $C_{12}H_{12}ClNO_2$
Calcd: C, 60.64; H, 5.09; N, 5.89.
Found: C, 60.48; H, 5.08; N, 5.84.

D. 3-(2-Chloro-4-cyanophenyl)propanoic acid

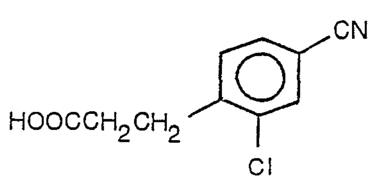

A mixture of ethyl 3-(chloro-4-cyanophenyl)propanoate (33.0 g, 0.14 mol), KOH (6.27 g, 0.11 mol) and 95% ethanol (109 mL) was warmed at 40°C for 1 h. The mixture was neutralized and evaporated to

dryness. The residue was dissolved in 5% $NaHCO_3$ and washed with EtOAc. Acidification and filtration gave crystalline 3-(2-chloro-4-cyanophenyl)propanoic acid: 18.4 g; mp 127—219°C.

E. 3-(2-Chloro-4-cyanophenyl)propanol

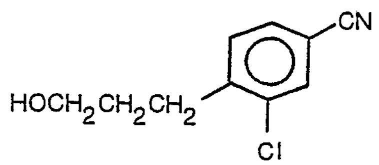

To 3-(2-chloro-4-cyanophenyl)propanoic acid (14.3 g, 0.068 mol) in 70 mL of dry THF at −18°C was added $B_2H_6$(72 ml, 0.068 mol) in THF. The mixture was stirred at room temperature overnight. After addition of water, the layers were separated and the aqueous phase was extracted with ether. Evaporation and distillation gave 3-(2-chloro-4-cyanophenyl)propanol as an oil: 11.0 g; bp 55—60°C (0.005 mm Hg).

Anal for $C_{10}H_{10}ClNO$:
Calcd: C, 61.39; H, 5.15; N, 7.16.
Found: C, 61.02; H, 5.19; N, 6.76.

F. 3-Chloro-4-(3-hydroxypropyl)propiophenone

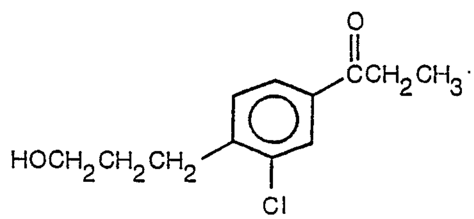

A mixture of 3-(2-chloro-4-cyanophenyl)propanol (18.0 g, 0.09 mol) and ethyl magnesium bromide (36.8 g, 0.276 mol) in dry ethyl ether was refluxed for 4 h. After quenching the reaction with 150 mL of 15% HCl, the layers were separated and the aqueous layer was extracted with ethyl acetate. Evaporation and recrystallization from ethyl ether-pentane gave 3-chloro-4-(3-hydroxpropyl)-propiophenone: 7.9 g; mp 46—48°C.

G. *α-t*-Butylamino-3-chloro-4-(3-hydroxypropyl)propiophenone hydrochloride three-fourths hydrate

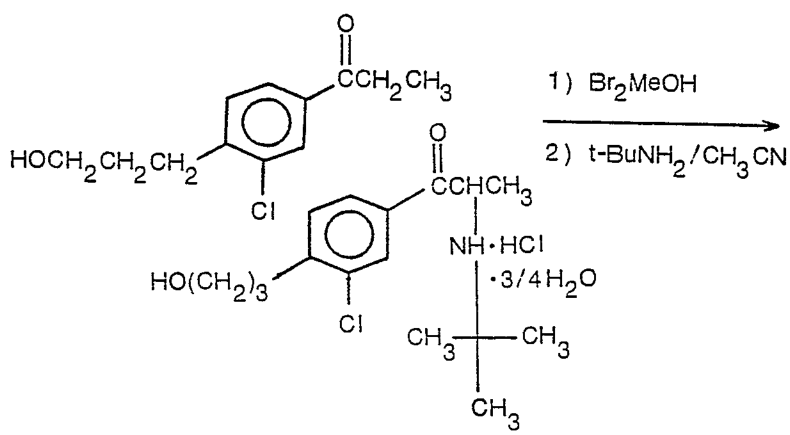


A mixture of 3-chloro-4-(3-hydroxypropyl)propiophenone (7.7 g, 0.03 mol) and bromine (5.7 g, 0.04 mol) in dry methanol was stirred at room temperature overnight. Evaporation and distillation gave *α*-bromo-3-chloro-4-(3-hydroxypropyl)propiophenone: 9.6 g; bp 102—105°C (0.005 mm Hg). The bromoketone was stirred overnight with a excess of tert-butylamine in $CH_3CN$ at room temperature. The crude *α-t*-butylamino-3-chloro-4-(3-hydroxypropyl)propiophenone was isolated and converted to hydrochloride salt: yield 1.25 g; mp 203—205°C ded.

Anal for $C_{16}H_{24}ClNO_2 \cdot HCl \cdot 3/4H_2O$;
Calcd: C, 55.22; H, 7.68; N, 4.02.
Found: C, 55.14; H, 7.39; N, 4.16.

H. α-*t*-Butylamino-3-chloro-4-(3-hydroxypropyl)propiophenone hemisuccinate (Compound 3)

α-*t*-Butylamino-3-chloro-4-(3-hydroxypropyl)propiophenone (100 mg; 0.288 mmole) and succinic anhydride (31.7 mg) were dissolved in pyridine (2.5 ml); the resulting solution was allowed to stand at room temperature overnight. Benzene (10 ml) was added and then evaporated under reduced pressure; this step was repeated four more times, giving a brown oil. The oil was dissolved in methanol (0.5 ml), and the solution was applied to two 0.5 mm silica gel plates (20 cm × 20 cm). The plates were developed using chloroform/methanol/aqueous ammonia (80/20/1) as the mobile phase. The silica gel strip containing the product (located by uv) was scraped off and the product extracted with chloroform/methanol (60/40; 2 × 100 ml). The combined extract was filtered and the solvent removed under reduced pressure. Ethyl acetate (30 ml) was added and the solution filtered. Evaporation of solvent gave α-*t*-butylamino-3-chloro-4-(3-hydroxypropyl)propiophenone hemisuccinate (25.4 mg) as a yellow oil. Analysis:1) thin layer chromatography: Rf = 0.15 on silica gel (0.25 mm) plates developed in chloroform/methanol/aqueous ammonia (80/20/1); 2) uv (in isotonic saline):$\lambda$max = 261 nm, $\lambda$min = 231 nm; nmr and mass spectrometry data were consistent with the assigned structure.

### Example III
### Immunogen Preparation

α-*t*-Butylamino-3-chloro-4-carboxymethoxypropiophenone-BSA (Compound 2-BSA)

Compound 2 (25.4 mg; 0.088 mmole) (obtained by basic hydrolysis of the corresponding methyl ester obtained in Example 1) was reacted with BSA (40 mg; 0.00057 mmole) and 1-ethyl-3-(3-dimethylaminopropyl)-carbodiimide (EDC) (25 mg) in 15 ml 10% dioxane/water at pH 5.7 overnight at 25°C. The reaction mixture was pressure dialyzed *vs.* deionized water, concentrated by ultrafiltration and the immunogen lyophilized overnight.

### Example IV

α-*t*-Butylamino-3-chloro-4-γ-hydroxypropylpropiophenone hemisuccinate-BSA (Compound 3-BSA)

Compound 3 (25.5 mg; 0.064 mmole) reacted with triethylamine (TEA, 9.8 mg; 0.097 mmole) and isobutylchloroformate (9.8 mg; 0.072 mmole) in 1 ml dimethylformamide (DMF) at 4°C for 30 minutes to form the mixed anhydride. This reaction mixture was slowly added to a well-stirred solution of BSA (30 mg; 0.00043 mmole) in 4 ml of 0.1 M sodium carbonate at 4°C and allowed to react overnight. The reaction mixture was pressure dialyzed and lyophilized as in Example III.

### Example V

A. α-*t*-Butylamino-3-chloropropiophenone-carboxymethyloxime (Compound 1)

Bupropion (free base, 500 mg; 2.085 mmole), carboxymethoxylamine hemihydrochloride (1.37 g; 6.255 mmole) and anhydrous sodium acetate (684 mg; 8.34 mmole) in 50% aqueous ethanol (10 ml) were allowed to react at 25°C for 5 days. The pH was adjusted to 2.0 with 0.1 N HCl, and the mixture extracted with chloroform (3 × 20 ml). The combined extract was evaporated under reduced pressure, redissolved in chloroform (25 ml), evaporated under reduced pressure and redissolved in chloroform (2 ml). This solution was applied to eight 2 mm silica gel plates (20 cm × 20 cm) which were then developed with chloroform/methanol/aqueous ammonia (80/20/1). The band at Rf 0.2—0.3 was scraped off and extracted with ethyl acetate/methanol 90/10 (5×100 ml). The combined extract was filtered, evaporated under reduced pressure and dissolved in ethyl acetate (5 ml). This was filtered and evaporated under reduced pressure to give α-*t*-butylamino-3-chloropropiophenone-carboxymethyloxime as an oil (135 mg). Analysis: uv (in isotonic saline): $\lambda$max = 250 nm, $\lambda$min = 230 nm; nmr was consistent with the assigned structure.

B. β-*t*-Butylamino-3-chloropropionphenone-carboxymethyloxime-BSA, (Compound 1-BSA)

Compound 1 was coupled to bovine serum albumin (BSA) using a water-soluble carbodiimide to catalyze the formation of peptide bonds between the free acid moiety of II and the ε-amino groups of lysyl residues of BSA. Compound 1 (25 mg; 0.080 mmole) was reacted with BSA (40 mg; 0.00057 mmole), and 1-ethyl-3-(3-dimethylaminopropyl)-carbodiimide (EDC, 25 mg) in 15 ml 10% dioxane/water at pH 5.7 overnight at 25°C. The reaction mixture was pressure dialyzed *vs* deionized water, concentrated by ultra-filtration and the immunogen lyophilized overnight.

### Example VI

α-*t*-Butylamino-3-chloropropiophenone-carboxymethyloxime-$^{125}$I-tryamine amide, (Compound 6)

Compound 1 was coupled to tyramine using dicyclohexylcarbodiimide to catalyze the formation of a peptide bond between the free acid of II and the primary amino group of tyramine. Compound 1 (15 mg; 0048 mmole) reacted with tyramine (free base, 13 mg; 0.096 mmole) and dicyclohexylcarbodiimide DCC; 19 mg) in 2 ml dry dioxane at pH 9 overnight at 25°C. The product was purified by TLC, characterized spectrally and subsequently iodinated (Hunter-Greenwood Chloramine-T Method).

Example VII

α-*t*-Butylamino-3-chloro-4-carbomethoxypropiophenone-$^{125}$I-tyramine amide, (Compound 7)

Compound 2 was coupled to tyramine using the mixed anhydride method. Compound 2 (25 mg; 0.076 mmole) was reacted with TEA (9.8 mg; 0.097 mmole) and isobutyl chloroformate (9.8 mg; 0.072 mmole) in 1 ml DMF at 4°C for 30 minutes to form the mixed anhydride. The reaction mixture was slowly added to a well-stirred solution of tyramine (free base, 10 mg; 0.073 mmole) in 1 ml DMF at 4°C and allowed to react overnight to yield to phenolic product. The product was purified by TLC, characterized spectrally and subsequently iodinated as in Example VI.

Example VIII

α - *t* - butylamino - 3 - chloro - 4 - λ - hydroxypropylpropiophenonehemisuccinate - $^{125}$I - tryamine amide, compound 8

Compound 3 was coupled to tryamine using the mixed anhydride method of Example VII. This product was purified by TLC and subsequently iodinated as described in Example VI.

Example IX

$^{125}$I-α-*t*-butylamino-3-iodo-4-hydroxypropiophenone, compound 9

α-*t*-butylamino-4-methoxypropiophenone hydrochloride (20.6 mg; 0.076 mmole) was suspended in 1 ml dry methylene chloride and cooled to —80°C in an acetone-dry ice bath. Boron tribromide (41.9 mg; 0.167 mmole) was added, the reaction mixture was protected from moisture with a calcium chloride drying tube, and the reaction was allowed to proceed overnight, during which time the temperature rose to 25°C. The reaction mixture was shaken with one volume methylene chloride (containing 0.02% water impurity) to liberate the product. The product was purified by TLC and subsequently iodinated as described in Example VI.

Example X

Preparation of α-*t*-butylamino-3-chloro-2-tritiopropiophenone hydrochloride ($^{3}$H-bupropion). (Compound 10)

A. 2-(3-Chloro-2-tritiophenyl)-4,4-dimethyl-2-oxazoline

2-(3-Chlorophenyl)-4,4-dimethyl-2-oxazoline was prepared from 3-chlorobenzoic acid by reaction with thionyl chloride to give 3-chlorobenzoylchloride, reaction of this acid chloride with 2-amino-2-methylpropanol to give the amide and cyclization of the amide with thionyl chloride followed by neutralization with dilute sodium hydroxide to give 2-(3-chlorophenyl)-4,4-dimethyl-2-oxazoline.

The oxazoline (419.2 mg) in dry ether (15 ml) was cooled to —78°C. and *tert*-butyllithium (2.0 mmoles) in pentane (1.12 ml) was added under nitrogen with stirring. Stirring was continued at —78°C. for 4 1/2 hours. Solvent-free $T_2O$ (0.5 ml, 90% isotopic purity) was added, and the solvent was allowed to slowly warm to room temperature and stirred overnight. The solvent was dried over anhydrous magnesium sulfate and filtered, and the solvent was removed *in vacuo* to give an orange oil. Purification was achieved by column chromatography on silica (12.6 g) packed as a chloroform slurry. The crude reaction product was added to the column as a chloroform solution. Elution was with chloroformethyl acetate (3:1 V/V). Fractions containing the radiolabeled oxazoline were combined. Solvent removal *in vacuo* gave 2-(3-chloro-2-tritiophenyl)-4,4-dimethyl-2-oxazoline (334 mg, 79%) as a yellow oil. Nmr and mass spectra data confirmed the structure; the radiolabel was about 90% of theoretical.

B. 2-(3-Chloro-2-tritiophenyl)-4,4-dimethyl-2 -oxazoline N-Methylfluorosulfonate

To 2-(3-chloro-2-tritiophenyl)-4,4-dimethyl-2-oxazoline (670.7 mg) in dry benzene (7 ml) under nitrogen was added methane fluorosulfonate (1.4 g). The mixture was stirred overnight at room temperature under nitrogen. The white precipitate was collected under nitrogen, washed with benzene (1 × 10 ml) and ether (2 × 10 ml) and dried to give 2-(3-chloro-2-tritiophenyl)-4,4-dimethyl-2-oxazoline N-methylfluorosulfonate in greater than 90% yield.

C. 2-(3-Chloro-2-tritiophenyl)-2-ethyl-4,4-dimethyl-N-methyloxazolidine

To the dry 2-(3-chloro-2-tritiophenyl)-4,4-dimethyl-2-oxazoline N-methylfluorosulfonate from step C in dry THF (15 ml) under nitrogen was rapidly added ethylmagnesium chloride (8.0 mmoles) in ether (2.7 ml). The mixture was stirred for 1 hour at room temperature and then poured into an ice-water mixture (75 ml) with stirring. The resulting suspension was extracted with ether (1 × 100 ml followed by 1 × 50 ml). The combined ether extracts were washed with water (1 × 50 ml) and dried over anhydrous magnesium sulfate. Filtration and removal of solvent *in vacuo* gave 2-(3-chloro-2-tritiophenyl)-2-ethyl-4,4-dimethyl-N-methyloxazolidine (83% yield) as a yellow oil.

D. 3-Chloro-2-tritiopropiophenone

A mixture of the crude oxazolidine from step C (670.5 mg) and oxalic acid (475.6 mg) in water (10 ml) was heated at reflux for 1 hour and then cooled to room temperature. Additional water (15 ml) was added, and the product was extracted with ether (2 × 15 ml). The ether layer was washed with 5%

potassium bicarbonate solution (2 x 15 ml) and dried over anhydrous magnesium sulfate. Solvent was removed *in vacuo* to give 3-chloro-2-tritiopropiophenone (405 mg).

E. $\alpha$-*t*-Butylamino-3-chloro-2 -tritiopropiophenone hydrochloride

Following the procedure of U.S. patent No. 3,819,706 incorporated herein by reference, 3-chloro-2-tritiopropiophenone was brominated and the $\alpha$-bromo product with *tert*-butylamine to give $\alpha$-*t*-butylamino-3-chloro-2-tritiopropiophenone which was isolated as the hydrochloride (43% yield).

Example XI

RIA of Bupropion in Plasma Using $^3H$ Bupropion

Samples of six standard solutions of bupropion in blank plasma having concentrations of 0.39 to 50 ng/ml) are pipetted (0.1 ml each) in duplicate into 12 x 75 mm plastic tubes. Duplicate non-specific binding (no antibody) and maximum binding tubes (no unlabeled bupropion) receive 0.1 ml blank plasma. Spiked plasma controls at several concentrations of (e.g., 1.0, 10.0, and 100 ng/ml) and plasma samples for assay (diluted into the standard range with blank plasma if necessary) are assayed in duplicate. After being placed in an ice bath, all tubes receive (in order) 0.2 ml $^3H$ bupropion (416 pg. containing approximately $10^4$ cpm) and 0.7 ml anti-bupropion serum (raised to a ringfunctionalized immunogen according to the present invention) (at pre-determined dilutions such that approximately 40% of the $^3H$ bupropion added is bound), both in assay buffer (0.05 M phosphate-buffered isotonic saline, containing 0.01 M EDTA and 0.1% gelatin, pH 7.0). Non-specific binding tubes receive 0.7 ml assay buffer). Following incubation either at 25°C for 2 hours or overnight at 4°C, bound and free $^3H$ bupropion are separated by incubation for 10 min at 0°C with 0.5 ml ice-cold dextran-coated charcoal solution (0.25% dextran, 5 mg/ml charcoal, in assay buffer) followed by centrifugation for 10 minutes at 4°C (5000 rpm) to pellet the charcoal. The supernatants, containing antibody-bound $^3H$ bupropion are carefully decanted into liquid scintillation cocktail for quantitation of $^3H$. After counts (minus average non-specific binding cpm) are expressed as percentages of average maximum binding cpm, bupropion concentrations in plasma samples and spiked controls are read from a standard curve (log concentration *vs* percent average maximum binding).

Example XII

The procedure of Example XI has been applied to a study of bupropion (supplied as Wellbatrin, produced by Burroughs Wellcome Co.) pharmacokinetics in plasma after oral administration of 200 mg bupropion-HCl (in two 100 mg tablets) to four normal male subjects. Venous blood samples were collected at various times following dosing, and concentrations in plasma samples determined as described. The assay standard curve obtained by this method ($\pm$ interassay standard deviation, $N = 8$) is shown in Figure 1. Spiked controls (at 1, 10 and 100 ng/ml) run with 8 separate assays gave interassay values of $0.89 \pm 0.11$ (S.D.), $10.35 \pm 0.60$, and $92.72 \pm 5.02$, respectively. Plots of elapsed time *vs* plasma bupropion concentration for each subject were made. Using these data, various pharmacokinetic parameters were calculated using the C-strip and NONLIN computer programs. The mean elimination half-life, maximum plasma bupropion concentration and time to reach this maximum concentration were $2.9 \pm 0.4$ (S.D.) hours, $217 \pm 37$ ng/ml, and $1.5 \pm 0.4$ hours, respectively.

Examples XIII—XVII

RIA of Bupropion using $^{125}I$-labeled Bupropion Competitors

RIA's have been run according to the method of Example X utilizing $^{125}I$ labeled bupropion competitors of the present invention in conjunction with antisera obtained following immunization with immunocens of the present invention. Compound 6 was used in conjunction with anti-compound 1 sera. Compounds 7, 8 and 9 were each used with anti-compound 2 sera, and with anti-compound 3 sera. Following the ethanol precipitation of antibody-bound $^{125}I$-bupropion competitior at equilibrium, $^{125}I$ in the protein pellets was quantified. Figure 2 shows standard curves obtained with the various iodinated competitors.

It can be seen that compound 9, in conjunction with either antisera produces RIA's approximately ten times more sensitive than the compound 10 ($^3H$ bupropion) assay system. Substitution of 7 or 8 yields sensitivity approximately equal to that obtained with the compound 10 system. The anti-compound 1 sera and compound 6, which preferably are used for RIA only in conjunction with each other, produced the least sensitive RIA. However, by virtue of derivatization *via* the ketone, these antisera are believed to exhibit much less cross reaction with ring-hydroxylated metabolites, albeit at the expense of specificity for changes in structure at the ketone.

Example XVIII

Preparation of $\alpha$-t-butylamino-3-chloro-2-tritiopropiophenone hydrochloride (Compound 10)

A. *2-(3-Chloro-2-tritiophenyl)-4,4-dimethyl-2-oxazoline*

2-(3-Chlorophenyl)-4,4-dimethyl-2-oxazoline was prepared from 3-chlorobenzoic acid by reaction with thionyl chloride to give 3-chlorobenzoylchloride, reaction of this acid chloride with 2-amino-2-methylpropanol to give the amide and cyclization of the amide with thionyl chloride followed by

neutralization with dilute sodium hydroxide to give 2-(3-chlorophenyl)-4,4-dimethyl-2-oxazoline.

The oxazoline (419.2 mg) in dry ether (15 ml) was cooled to −78°C. and *tert*-butyllithium (2.0 m moles) in pentane (1.12 ml) was added under nitrogen with stirring. Stirring was continued at −78°C. for 4 1/2 hours. Solvent-free $T_2O$ (0.5 ml, 90% isotopic purity) was added, and the solution was allowed to slowly warm to room temperature and stirred overnight. The solution was dried over anhydrous magnesium sulfate and filtered, and the solvent was removed *in vacuo* to give an orange oil. Purification was achieved by column chromatography on silica (12.6 g) packed as a chloroform slurry. The crude reaction product was added to the column as a chloroform solution. Elution was with chloroformethyl acetate (3:1 V/V). Fractions containing the radiolabeled oxazoline were combined. Solvent removal *in vacuo* gave 2-(3-chloro-2-tritiophenyl)-4,4-dimethyl-2-oxazoline (334 mg, 79%) as a yellow oil. Nmr and mass spectral data confirmed the structure; the radiolabel was about 90% of theoretical.

B. *2-(3-Chloro-2-tritiophenyl)-4,4-dimethyl2-oxazoline N-Methylfluorosulfonate*

To 2-(3-Chloro-2-tritiophenyl)-4,4-dimethyl-2-oxazoline (670.7 mg) in dry benzene (7 ml) under nitrogen was added methane fluorosulfonate (1.4 g). The mixture was stirred overnight at room temperature under nitrogen. The white precipitate was collected under nitrogen, washed with benzene (1 × 10 ml) and ether (2 × 10 ml) and dried to give (2-(3-chloro-2-tritiophenyl)-4,4-dimethyl-2-oxazoline N-methylfluorosulfonate in greater than 90% yield.

C. *2-(3-Chloro-2-tritiophenyl)-2-ethyl-4,4-dimethyl-N-methyloxazolidine*

To the dry 2-(3-chloro-2-tritiophenyl)-4,4-dimethyl-2-oxazoline N-methylfluorosulfonate from step C in dry THF (15 ml) under nitrogen was rapidly added ethylmagnesium chloride (8.0 m moles) in ether (2.7 ml). The mixture was stirred for 1 hour at room temperature and then poured into an ice-water mixture (75 ml) with stirring. The resulting suspension was extracted with ether (1 × 100 ml followed by 1 × 50 ml). The combined ether extracts were washed with water (1 × 50 ml) and dried over anhydrous magnesium sulfate. Filtration and removal of solvent *in vacuo* gave 2 - (3 - chloro - 2 - tritiophenyl) - 2 - ethyl - 4,4 - dimethyl - N - methyloxazolidine (83% yield) as a yellow oil.

D *3-Chloro-2-tritiopropiophenone*

A mixture of the crude oxazolidine from step C (670.5 mg) and oxalic acid (475.6 mg) in water (10 ml) was heated at reflex for 1 hour and then cooled to room temperature. Additional water (15 ml) was added, and the product was extracted with ether (2 × 15 ml). The ether layer was washed with 5% potassium bicarbonate solution (2 × 15 ml) and dried over anhydrous magnesium sulfate. Solvent was *in vacuo* to give 3-chloro-2-tritiopropiophenone (405 mg).

E. *α-t-Butylamino-3-chloro-2-tritiopropiophenone hydrochloride*

Following the procedure of U.S. patent No. 3,819,706 incorporated herein by reference, 3-chloro-2-tritiopropiophenone was brominated and the α-bromo product treated with *tert*-butylamine to give α-*t*-butylamino-3-chloro-2-tritiopropiophenone which was isolated as the hydrochloride (43% yield).

## SYNTHESIS METHOD A

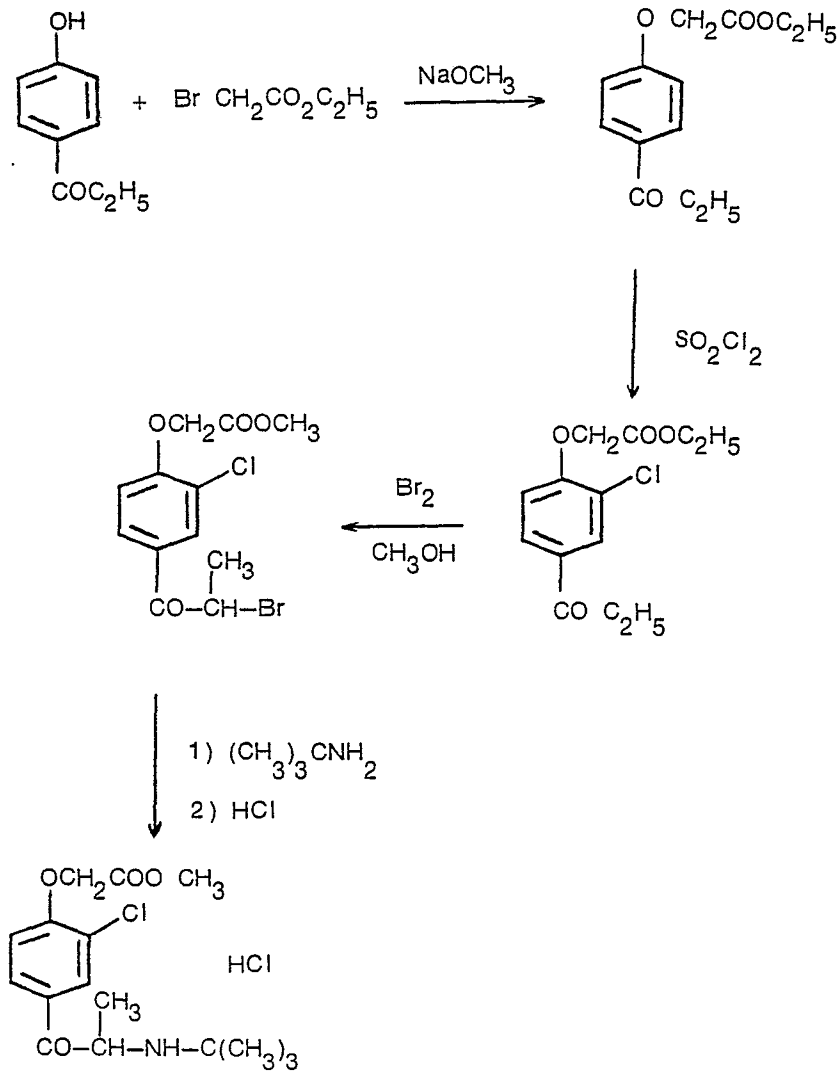


## Claims

1. A compound of the formula:

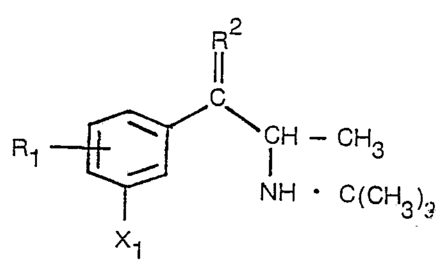

I

wherein
(1) $X_1$ is Cl, $R^2$ is oxygen and
$R^1$ is $-(CH_2)_n-OH$
or $-(CH_2)_n-O-(CO)_m(CH_2)_pCOZ$
or $-O-(CH_2)_pCOZ$
or $-O-(CH_2)_pCOOR_{11}$
or is a radioisotope

or

(2) $X_1$ is Cl, $R^1$ is H and $R^2$ is a $=N{\cdot}O(CH_2)_qCOZ$

or

(3) $X_1$ is a radioisotope, $R_2$ is oxygen and $R_1$ is OH in the 4-position

and wherein n is an integer of 1—5, m is 0 or 1, p is an integer of 1—4, q is an integer of 1—3, $R_{11}$ is a $C_1$—$C_3$ alkyl group and Z is OH or a group of formula:

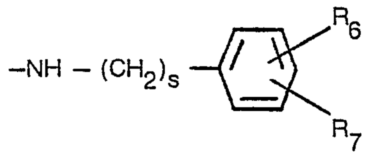

or

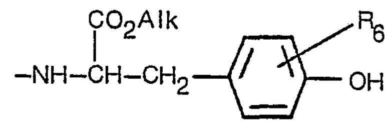

where $R_6$ is a radioactive nuclide, $R_7$ is OH, Alk is alkyl of 1 to 4 carbon atoms and s is an integer of 1—4.

2. A compound according to claim 1 wherein $X_1$ is Cl, $R^2$ is oxygen and $R_1$ is in the 4-position.

3. A compound according to claim 1 or 2 wherein $X_1$ is Cl, $R^2$ is oxygen and $R_1$ is $—(CH_2)_3 \cdot OCO(CH_2)_2COOH$ or $—(CH_2)_3OH$.

4. A compound according to claim 1 or 2 wherein $X_1$ is Cl, $R^2$ is oxygen and $R_1$ is

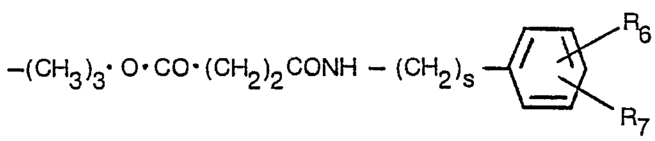

where s, $R_6$ and $R_7$ are as defined in claim 1.

5. A compound according to claim 1 or 2 wherein $X_1$ is Cl, $R^2$ is oxygen and $R_1$ is $—OCH_2—COOH$ or $—OCH_2COOCH_3$.

6. A compound according to claim 1 or 2 wherein $X_1$ is Cl, $R^2$ is oxygen and $R_1$ is

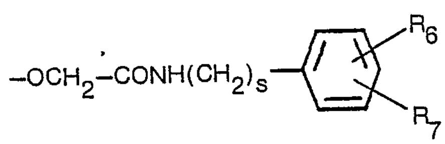

where s, $R_6$ and $R_7$ are as defined in claim 1.

7. A compound according to claim 1 wherein $X_1$ is Cl, $R^1$ is H and $R^2$ is $=N—O—CH_2COOH$.

8. A compound according to claim 1 wherein $X_1$ is Cl, $R^1$ is H and $R^2$ is

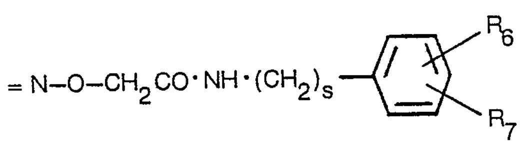

wherein s, $R_6$ and $R_7$ are as defined in claim 1.

9. A compound according to claim 4, 6 or 8 wherein $R_6$ is $^3H$ or $^{127}I$.

10. A compound according to claim 9 wherein $R_6$ is $^{127}I$, $R_7$ is 4—OH and s is 2.

11. A compound according to claim 1 wherein $X_1$ is Cl, $R^2$ is oxygen and $R^1$ is 3H or a radioisotope of iodine.

12. A compound according to claim 11 wherein $R^1$ is at the 2-position.

13. A compound according to claim 1 wherein $R^2$ is oxygen, $R^1$ is a 4—OH and $X_1$ is $^3H$ or a radioisotope of iodine.

14. A compound according to any one of claims 11—13 wherein the radioisotope of iodine is $^{125}I$.

15. A process for preparation of a compound of formula I as defined in claim 1 in which $X_1$ is Cl, $R^2$ is oxygen and $R_1$ is $—(CH_2)_n—OCO(CH_2)_pCOOH$ which comprises reacting a compound of formula I in which $X_1$ is Cl, $R^2$ is oxygen and $R_1$ is $—(CH_2)_n—OH$ with an anhydride of formula

$OCO(CH_2)_pCO.$

16. A process for the preparation of a compound of formula I as defined in claim 1 in which $X_1$ is Cl, $R^2$ is oxygen and $R_1$ is $—O(CH_2)_pCOOH$ which comprises subjecting to basic hydrolysis a compound of formula I as defined in claim 1 in which $X_1$ is Cl, $R^2$ is oxygen and $R_1$ is $—O(CH_2)_pCOOR_{11}$.

17. A process for the preparation of a compound of formula I as defined in claim 1 in which $X_1$ is Cl, $R^1$ is H and $R^2$ is $=N{\cdot}O(CH_2)_qCOOH$ which comprises reacting bupropion with a carboxyalkoxylamine hemihydrochloride containing 1—3 carbon atoms in the alkoxy residue.

18. A process for the preparation of a compound of formula I as defined in claim 1 in which $X_1$ is Cl, $R^2$ is oxygen and $R^1$ is $—(CH_2)_n—O{\cdot}CO(CH_2)_p—COZ$ or $—O—(CH_2)_pCOZ$ where Z is

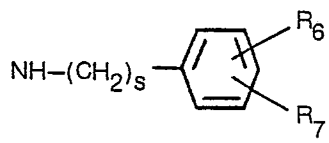

which comprises reacting a compound of formula I as defined in claim 1 in which $X_1$ is Cl, $R^2$ is oxygen and $R^1$ is $—(CH_2)_n—OCO(CH_2)_pCOOH$ or $—O—(CH_2)_pCOOH$ with an alkyl haloformate (containing 1—4 carbon atoms in the alkyl group) in the presence of a trialkylamine, reacting the resulting reaction product with an amine of formula:

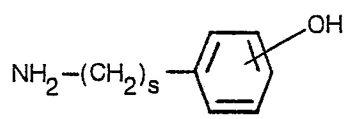

and then introducing the radioactive nuclide into the ring originating from the amine.

19. A process according to claim 18 wherein the haloformate is isobutyl chloroformate, the trialkylamine is triethylamine and the amine is tyramine.

20. A process for preparing a compound of formula I as defined in claim 1 in which $X^1$ is Cl, $R^1$ is H and $R^2$ is

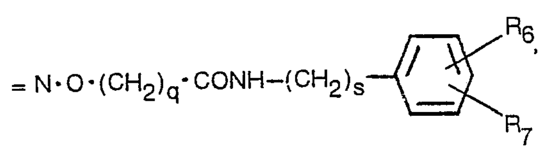

which comprises reacting a compound of formula I as defined in claim 1 in which $X^1$ is Cl, $R^1$ is H and $R^2$ is $=N{\cdot}O{\cdot}(CH_2)_qCOOH$ with an amine of formula:

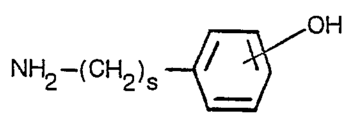

and subsequently introducing the radioactive nuclide into the ring originating from the amine.

21. A process according to claim 20 wherein the reaction between the acid and amine is carried out in the presence of dicyclohexylcarbodiimide.

22. A process according to claim 20 or 21 wherein the amine is tyramine.

23. An immunogen comprising a conjugate of an immunogenic carrier material and a compound according to any one of claims 1—5 or 7 in which $R^1$ is $—(CH_2)_n—O—(CO)_m—(CH_2)_pCOOH$ or $—O—(CH_2)_pCOOH$ or $R^2$ is $=N{\cdot}O{\cdot}(CH_2)_qCOOH$.

24. An immunogen according to claim 23 wherein the carrier material is bovine serum albumen.

25. A method of preparing an immunogen as defined in claim 23 or 24 which comprises reacting an immunogenic carrier material with a compound according to any one of claims 1—5 or 7 in which $R^1$ is $—(CH_2)_n—O—(CO)_m{\cdot}(CH_2)_pCOOH$ or $—O—(CH_2)_pCOOH$ or $R^2$ is $=N{\cdot}O{\cdot}(CH_2)_pCOOH$ in the presence of a coupling agent.

26. A method of producing an anti-serum containing antibodies to bupropion and active metabolites thereof which comprises presenting to the immune response system of a host animal an immunogen according to claim 23 or 24 and recovering serum containing antibodies from the blood of the host animal.

27. A method according to claim 26 wherein the host animal is rabbit.

28. A method of determining the concentration of bupropion and any active metabolites thereof in a body fluid which comprises

(a) mixing the body fluid with (1) an anti-serum obtained by a method according to claim 26 or 27 and (2) a labelled competitor of bupropion which is a compound of formula I as defined in any one of claims 1, 2, 4, 6 or 8—14 containing a radioactive nuclide and which is capable of competing with bupropion for binding to the anti-serum; and

(b) mixing together a sample containing a known concentration of bupropion with an anti-serum (1) and a labelled competitor (2); and

(c) measuring the amount of labelled competitor either bound or unbound to the anti-serum in each of step (a) and step (b) and comparing the result for step (a) with the corresponding result for step (b).

29. A method according to claim 28 in which the competitor, antisera and body fluid are combined in the presence of sufficient buffer to produce a pH of about 6 to 9.

30. A method according to claim 28 or 29 in which unbound antiserum and body fluid are removed as part of the measurement.

31. A method according to any one of claims 28—30 in which the body fluid is blood plasma or blood serum.

32. A method according to any one of claims 28—31 in which step (b) is repeated a sufficient number of times while varying the known concentration of bupropion to provide information for generating a standard curve for interpolating the amount of bupropion and any active metabolites thereof in the body fluid.

33. A method according to any one of claims 28—32 in which the concentration of bupropion and active metabolite is determined by reference to a standard curve representing per cent inhibition of the binding of competitor to the antisera by bupropion.

34. A method according to any one of claims 28—33 in which the concentration of body fluid is less than about 10%.

35. A method according to any one of claims 28—34 in which the amount of body fluid in the mixture containing same is greater than one microlitre.

36. A method according to any one of claims 28—35 wherein the body fluid is dialyzed prior to mixing.

37. A method according to any one of claims 28—36 in which the unbound bupropion and body fluid are removed prior to determining the per cent inhibition of binding.

38. A method according to any one of claims 28—37 wherein, in step (c), the antisera is precipitated by adding ice cold ethanol to the mixture prior to making the measurement.

39. A method according to any one of claims 28—38 wherein the competitor is $\alpha$ - *t* - butylamino - 3 - chloropropiophenone-carboxymethyloxime-$^{125}$I-tyramine amide; or $^{125}$I - $\alpha$ - *t* - butylamino - 3 - iodo - 4 - hydroxypropiophenone; or $\alpha$ - *t* - butylamino - 3 - chloro - 6 - [$^{3}$H] - propiophenone hydrochloride;
or

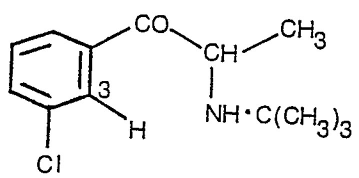

40. A method according to any one of claims 28—39 wherein the antiserum is one raised using $\alpha$ - *t* - butylamino - 3 - chloropropiophenone - carboxymethyloxime/bovine serum albumin conjugate as immunogen and the competitor is $\alpha$ - *t* - butylamino - 3 - chloropropiophenone-carboxymethyloxime - $^{125}$I - tyramine amide.

41. A test kit comprising a first component which is an anti-serum obtained by a method according to claim 26 or 27 and a second separately packaged component which is a labelled competitor for bupropion which is a compound of formula I as defined in any one of claims 1, 2, 4, 6 or 8—14 containing a radioactive nuclide.

42. A test kit according to claim 41 containing a third separately packaged component which is bupropion.

43. A test kit according to claim 41 or 42 wherein the anti-serum is one raised using $\alpha$ - *t* - butylamino - 3 - chloropropiophenone - carboxymethyloxime/bovine serum albumin conjugate as immunogen and the competitor is $\alpha$ - *t* - butylamino - 3 - chloropropiophenone - carboxymethyloxime - $^{125}$I - tyramine amide.

**Patentansprüche**

1. Verbindung der Formel I

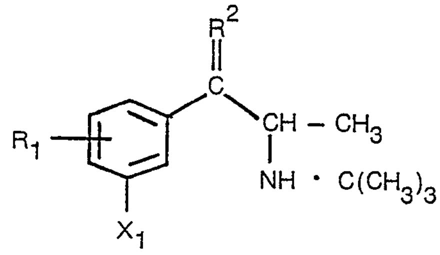

I

worin

(1) $X_1$ für Cl steht, $R^2$ Sauerstoff ist und $R_1$ der Rest $-(CH_2)_n-OH$ oder $-(CH_2)_n-O-(CO)_m(CH_2)_pCOZ$ oder $-O-(CH_2)_pCOZ$ oder $-O-(CH_2)_pCOOR_{11}$ oder ein Radioisotop ist

oder

(2) $X_1$ für Cl steht, $R_1$ ein Wasserstoffatom darstellt und $R^2$ den Rest $=N{\cdot}O(CH_2)_qCOZ$ bedeutet

oder

(3) $X_1$ ein Radioisotop ist, $R_2$ ein Sauerstoffatom darstellt und $R_1$ ein OH-Gruppe in der 4-Stellung bedeutet

und worin n eine ganze Zahl von 1—5 darstellt, m gleich 0 oder 1 ist, p eine ganze Zahl von 1—4 bedeutet, q eine ganze Zahl von 1—3 ist, $R_{11}$ einen $C_1$—$C_3$-Alkylrest darstellt und Z eine OH-Gruppe oder einen Rest der Formel

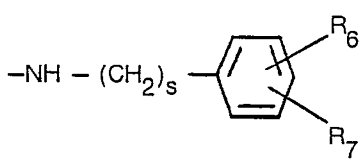

oder

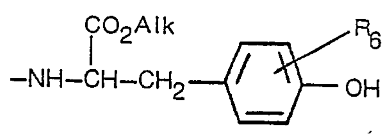

bedeutet, worin $R_6$ ein radioaktives Nuklid darstellt, $R_7$ eine OH-Gruppe bedeutet, Alk für einen Alkylrest mit 1 bis 4 Kohlenstoffatomen steht und s eine ganze Zahl von 1 bis 4 ist.

2. Verbindung nach Anspruch 1, worin $X_1$ gleich Cl ist, $R_2$ ein Sauerstoffatom darstellt und $R_1$ sich in der 4-Stellung befindet.

3. Verbindung nach Anspruch 1 oder Anspruch 2, worin $X_1$ für Cl steht, $R^2$ ein Sauerstoffatom ist und $R_1$ den Rest $-(CH_2)_3{\cdot}OCO-(CH_2)_2COOH$ oder $-(CH_2)_3OH$ bedeutet.

4. Verbindung nach Anspruch 1 oder Anspruch 2, worin $X_1$ für Cl steht, $R^2$ ein Sauerstoffatom darstellt und $R_1$ den Rest

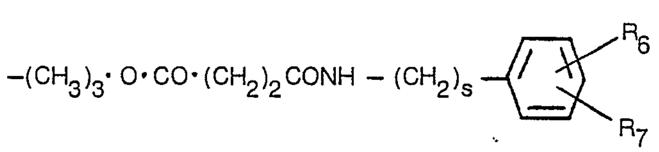

darstellt, worin s, $R_6$ und $R_7$ die in Anspruch 1 angegebene Bedeutung haben.

5. Verbindung nach Anspruch 1 oder Anspruch 2, worin $X_1$ für Cl steht, $R^2$ ein Sauerstoffatom bedeutet und $R_1$ den Rest $-OCH_2-COOH$ oder $-OCH_2COOCH_3$ bedeutet.

6. Verbindung nach Anspruch 1 oder 2, worin $X_1$ für Cl steht, $R^2$ ein Sauerstoffatom bedeutet und $R_1$ den Rest

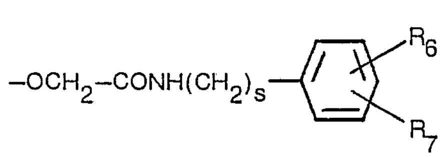

darstellt, worin s, $R_6$ und $R_7$ die in Anspruch 1 angegebene Bedeutung besitzen.

7. Verbindung nach Anspruch 1, worin $X_1$ für Cl steht, $R^1$ ein Wasserstoffatom bedeutet und $R^2$ den Rest $=N-O-CH_2COOH$ darstellt.

8. Verbindung nach Anspruch 1, worin $X_1$ für Cl steht, $R^1$ ein Wasserstoffatom bedeutet und $R^2$ den Rest

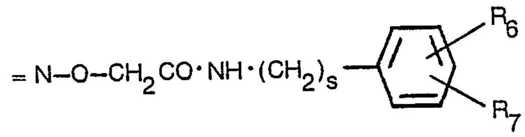

darstellt, worin s, $R_6$ und $R_7$ die in Anspruch 1 angegebene Bedeutung haben.

9. Verbindung nach Anspruch 4, 6 oder 8, worin $R_6$ für $^3H$ oder $^{127}J$ steht.

10. Verbindung nach Anspruch 9, worin $R_6$ für $^{127}J$ steht, $R_7$ gleich $4-OH$ ist und s den Wert 2 hat.

11. Verbindung nach Anspruch 1, worin $X_1$ für Cl steht, $R^2$ ein Sauerstoffatom bedeutet und $R^1$ gleich $^3H$ oder ein Radioisotop von Jod ist.

12. Verbindung nach Anspruch 11, worin $R^1$ sich in der 2-Stellung befindet.

13. Verbindung nach Anspruch 1, worin $R^2$ ein Sauerstoffatom darstellt, $R^1$ eine Gruppe $4-OH$ ist und $X_1$ für $^3H$ oder ein Radioisotop von Jod steht.

14. Verbindung nach einem der Ansprüche 11 bis 13, worin das Radioisotop des Jods gleich $^{125}J$ ist.

15. Verfahren zur Herstellung einer Verbindung nach Formel I gemäß der Definition in Anspruch 1, worin $X_1$ für Cl steht, $R^2$ ein Sauerstoffatom bedeutet und $R_1$ den Rest $-(CH_2)_n-OCO(CH_2)_pCOOH$ bedeutet, wobei eine Verbindung der Formel I, in der $X_1$ für Cl steht, $R^2$ ein Sauerstoffatom bedeutet und $R_1$ den Rest $-(CH_2)_n-OH$ darstellt, mit einem Anhydrid der Formel

$$OCO(CH_2)_pCO$$

umgesetzt wird.

16. Verfahren zur Herstellung einer Verbindung der Formel I gemäß der Definition in Anspruch 1, worin $X_1$ für Cl steht, $R^2$ ein Sauerstoffatom darstellt und $R_1$ den Rest $-O(CH_2)_pCOOH$ bedeutet, wobei eine Verbindung der Formel I gemäß der Definition in Anspruch 1, worin $X_1$ für Cl steht, $R^2$ ein Sauerstoffatom bedeutet und $R_1$ den Rest $-O(CH_2)_pCOOR_{11}$ darstellt, einer basischen Hydrolyse unterworfen wird.

17. Verfahren zur Herstellung einer Verbindung der Formel I gemäß der Definition in Anspruch 1, worin $X_1$ für Cl steht, $R^1$ ein Wasserstoffatom bedeutet und $R^2$ den Rest $=N{\cdot}O(CH_2)_qCOOH$ bedeutet, worin Bupropion mit einem Carboxyalkoxylamin-hemihydrochlorid, welches 1 bis 3 Kohlenstoffatome in dem Alkoxyrest enthält, umgesetzt wird.

18. Verfahren zur Herstellung einer Verbindung der Formel I gemäß der Definition in Anspruch 1, worin $X_1$ für Cl steht, $R^2$ ein Sauerstoffatom bedeutet und $R^1$ den Rest $-(CH_2)_n-O{\cdot}CO(CH_2)_p-COZ$ oder $-O-(CH_2)_pCOZ$ darstellt, worin Z für

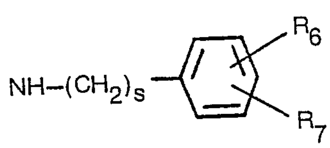

steht, wobei eine Verbindung der Formel I gemäß der Definition in Anspruch 1, worin $X_1$ für Cl steht, $R^2$ ein Sauerstoffatom bedeutet und $R^1$ den Rest $-(CH_2)_n-OCO(CH_2)_pCOOH$ oder $-O-(CH_2)_pCOOH$ bedeutet, mit einem Alkylhalogenformiat (welches 1 bis 4 Kohlenstoffatome in der Alkylgruppe enthält) in der Gegenwart eines Trialkylamins umgesetzt wird, das sich ergebende Reaktionsprodukt mit einem Amin der Formel

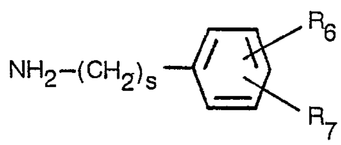

umgesetzt wird, und sodann das radioaktive Nuklid in den Ring eingeführt wird, der vom Amin herrührt.

19. Verfahren nach Anspruch 18, worin das Halogenformiat das Isobutylchlorformiat ist, das Trialkylamin das Triethylamin ist und das Amin gleich Tyramin ist.

20. Verfahren zur Herstellung einer Verbindung der Formel I gemäß der Definition in Anspruch 1, worin $X_1$ für Cl steht, $R^1$ ein Wasserstoffatom bedeutet und $R^2$ den Rest

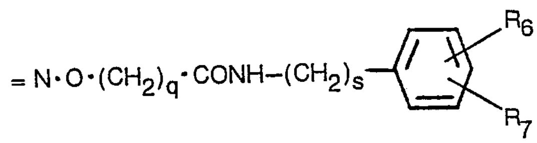

darstellt, wobei eine Verbindung der Formel I gemäß der Definition in Anspruch 1, worin $X^1$ für Cl steht, $R^1$ ein Wasserstoffatom bedeutet und $R^2$ den Rest $=N\cdot O\cdot(CH_2)_qCOOH$ darstellt, mit einem Amin der Formel

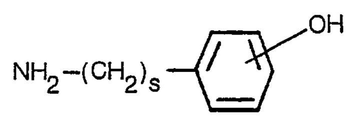

umgesetzt wird und anschließend das radioaktive Nuklid in den Ring, der vom Amin herrührt, eingeführt wird.

21. Verfahren nach Anspruch 20, worin die Reaktion zwischen der Säure und dem Amin in Gegenwart von Dicyclohexylcarbodiimid durchgeführt wird.

22. Verfahren nach Anspruch 20 oder 21, worin das Amin das Tyramin ist.

23. Immunogen, enthaltend ein Konjugat eines immunogenen Trägermaterials und eine Verbindung nach einem der Ansprüche 1 bis 5 oder 7, worin $R^1$ den Rest $-(CH_2)_n-O-(CO)_m-(CH_2)_pCOOH$ oder $-O-(CH_2)_pCOOH$ bedeutet oder $R^2$ den Rest $=N\cdot O\cdot(CH_2)_qCOOH$ darstellt.

24. Immunogen nach Anspruch 23, worin das Trägermaterial Rinder-Serumalbumin ist.

25. Verfahren zur Herstellung eines Immunogens gemäß der Definition in Anspruch 23 oder 24, wobei ein immunogenes Trägermaterial mit einer Verbindung nach einem der Ansprüche 1 bis 5 oder 7, worin $R^1$ den Rest $-(CH_2)_n-O-(CO)_m\cdot(CH_2)_pCOOH$ oder $-O-(CH_2)_pCOOH$ bedeutet oder $R^2$ den Rest $=N\cdot O\cdot(CH_2)_pCOOH$ darstellt, in Gegenwart eines Kupplungsmittels umgesetzt wird.

26. Verfahren zur Herstellung eines Antiserums, welches Antikörper gegenüber Bupropion und aktiven Metaboliten davon enthält, wobei gegenüber dem Immun-Ansprechsystem eines Wirtstieres ein Immunogen nach Anspruch 23 oder 24 gegenübergestellt wird und das Serum, welches Antikörper aus dem Blut des Wirtstieres enthält, gewonnen wird.

27. Verfahren nach Anspruch 26, worin das Wirtstier ein Kaninchen ist.

28. Verfahren zur Bestimmung der Konzentration von Bupropion und eines beliebigen aktiven Metaboliten davon in einer Körperflüssigkeit, wobei

(a) die Körperflüssigkeit mit (1) einem Antiserum, welches nach einem Verfahren gemäß Anspruch 26 oder 27 erhalten worden ist und (2) einer markierten Konkurrenzverbindung von Bupropion, welche eine Verbindung der Formel I gemäß der Definition in einem der Ansprüche 1, 2, 4, 6 oder 8 bis 14 ist, welche ein radioaktives Nuklid enthält und in der Lage ist, mit Bupropion um die Bindung an das Antiserum zu konkurrieren, vermischt wird, und

(b) eine Probe, welche eine bekannte Konzentration an Bupropion enthält, mit einem Antserum (1) und einer markierten Konkurrenzverbindung (2) vermischt wird, und

(c) die Menge der markierten Konkurrenzverbindung, die entweder gebunden oder ungebunden gegenüber dem Antiserum vorliegt, in jedem der beiden Schritte (a) und (b) gemessen wird und das Ergebnis für Schritt (a) mit dem entsprechenden Ergebnis für Schritt (b) verglichen wird.

29. Verfahren nach Anspruch 28, worin die Konkurrenzverbindung, Antisera und Körperflüssigkeit in Gegenwart eines hinreichenden Puffers, um einen pH-Wert von etwa 6 bis 9 einzustellen, vereint werden.

30. Verfahren nach Anspruch 28 oder 29, worin ungebundenes Antiserum und Körperflüssigkeit als Teil des Meßvorgangs entfernt werden.

31. Verfahren nach einem der Ansprüche 28 bis 30, worin die Körperflüssigkeit Blutplasma oder Blutserum ist.

32. Verfahren nach einem der Ansprüche 28 bis 31, worin Schritt (b) hinreichende Male wiederholt wird, während die bekannte Konzentration von Bupropion variiert wird, um Informationen zu geben zur Herstellung einer Standardkurve für die Interpolation der Menge an Bupropion und beliebiger aktiver Metaboliten davon in der Körperflüssigkeit.

33. Verfahren nach einem der Ansprüche 28 bis 32, worin die Konzentration an Bupropion und aktivem Metaboliten durch Referenz zu einer Standardkurve bestimmt wird, welche die prozentuale Inhibition der Bindung der Konkurrenzsubstanz zum Antiserum durch Bupropion angibt.

34. Verfahren nach einem der Ansprüche 28 bis 33, worin die Konzentration an Körperflüssigkeit weniger als etwa 10% beträgt.

35. Verfahren nach einem der Ansprüche 28 bis 34, worin die Menge an Körperflüssigkeit in dem Gemisch, welches diese enthält, größer als 1 Mikroliter ist.

36. Verfahren nach einem der Ansprüche 28 bis 35, worin die Körperflüssigkeit vor dem Vermischen dialysiert wird.

37. Verfahren nach einem der Ansprüche 28 bis 36, worin das ungebundene Bupropion und die

Körperflüssigkeit vor der Bestimmung der prozentualen Inhibition der Bindung entfernt werden.

38. Verfahren nach einem der Ansprüche 28 bis 37, worin in Schritt (c) das Antiserum durch Zugabe von eiskaltem Ethanol zu dem Gemisch vor der Durchführung der Bestimmung niedergeschlagen wird.

39. Verfahren nach einem der Ansprüche 28 bis 38, worin die Konkurrenzverbindung $\alpha$ - tert. - Butylamino - 3 - chlorpropiophenon - carboxymethyloxim - $^{125}J$ - tyraminamid oder $^{125}J$ - $\alpha$ - tert. - Butylamino - 3 - jod - 4 - hydroxypropiophenon oder $\alpha$ - tert. - Butylamino - 3 - chlor - 6 - [$^3H$] - propiophenonhydrochlorid oder

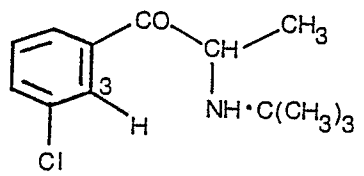

ist.

40. Verfahren nach einem der Ansprüche 28 bis 39, worin das Antiserum ein Antiserum ist, welches unter Verwendung von $\alpha$ - tert. - Butylamino - 3 - chlorpropiophenon - carboxymethyloxim/Rinderserumalbumin - Konjugat als Immunogen erzeugt worden ist und die Konkurrenzverbindung $\alpha$ - tert. - Butylamino - 3 - chlorpropiophenon - carboxymethyloxim - $^{125}J$ - tyraminamid ist.

41. Testsatz, enthaltend eine erste Komponente, die ein Antiserum ist, welches durch ein Verfahren nach Anspruch 26 oder 27 erhalten wurde, und eine zweite getrennt verpackte Komponente, die eine markierte Konkurrenzverbindung für Bupropion ist, welche eine Verbindung der Formel I gemäß der Definition in einem der Ansprüche 1, 2, 4, 6 oder 8 bis 14 ist, die ein radioaktives Nuklid enthält.

42. Testsatz nach Anspruch 41, enthaltend eine dritte getrennt verpackte Komponente, die Bupropion ist.

43. Testsatz nach Anspruch 41 oder 42, worin das Antiserum ein Antiserum ist, welches unter Verwendung von $\alpha$ - tert. - Butylamino - 3 - chlorpropiophenone - carboxymethyloxim/Rinderserumalbumin - Konjugat als Immunogen hergestellt wurde und die Konkurrenzsubstanz $\alpha$ - tert. - Butylamino - 3 - chlorpropiophenon - carboxymethyloxim - $^{125}J$ - tyraminamid ist.

## Revendications

1. Les composés de formule générale

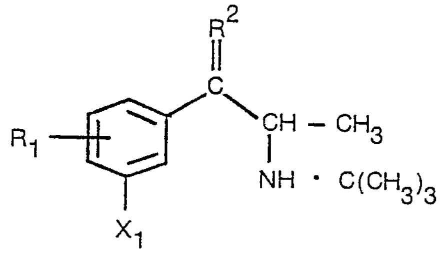

I

dans laquelle (1) $X_1$ représente Cl, $R^2$ l'oxygène et

$R_1$ und groupe

$—(CH_2)_n—OH$

$—(CH_2)_n—O—(CO)_m(CH_2)_pCOZ$

$—O—(CH_2)_pCOZ$

$—O—(CH_2)_pCOOR_{11}$

ou un isotope radioactif,

ou bien (2) $X_1$ représente Cl, $R^1$ l'hydrogène et

$R^2$ un groupe $=N{\cdot}O(CH_2)_qCOZ$,

ou encore (3) $X_1$ représente un radio-isotope, $R^2$ l'oxygène et $R^1$ un groupe OH à la position 4, n est un entier de 1 à 5, m le nombre 0 ou 1, p est un entier de 1 à 4, q un entier de 1 à 3, $R_{11}$ un alkyle en $C_1—C_3$ et Z un groupe OH ou un groupe

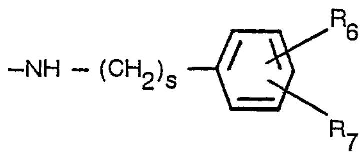

ou

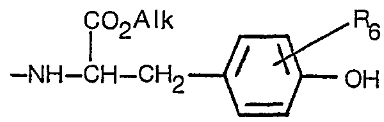

$R^6$ désignant un nuclide radioactif, $R^7$ un groupe OH, Alk un alkyle en $C_1$—$C_4$ et s un entier de 1 à 4.

2. Composé selon la revendication 1 dans lequel $X_1$ est un atome de chlore, $R_2$ un atome d'oxygène et $R_1$ occupe la position 4.

3. Composé selon la revendication 1 ou 2 dans lequel $X_1$ est un atome de chlore, $R_2$ est l'oxygène et $R_1$ un groupe $—(CH_2)_3{\cdot}OCO—(CH_2)_2COOH$ ou $—(CH_2)_3OH$.

4. Composé selon la revendication 1 ou 2 dans lequel $X_1$ est un atome de chlore, $R_2$ un atome d'oxygène et $R_1$ un groupe

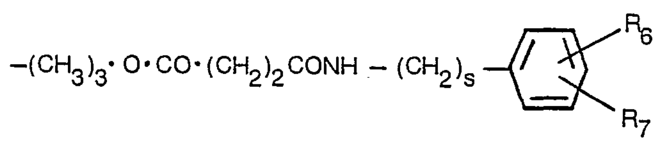

s, $R_6$ et $R_7$ ayant les significations données à la revendication 1.

5. Composé selon la revendication 1 ou 2, dans lequel $X_1$ est un atome de chlore, $R_2$ un atome d'oxygène, et $R_1$ un groupe $—OCH_2—COOH$ ou $—OCH_2COOCH_3$.

6. Composé selon la revendication 1 ou 2 dans lequel $X_1$ est un atome de chlore, $R_2$ un atome d'oxygène et $R_1$ un groupe

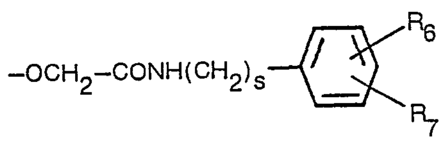

7. Composé selon la revendication 1 dans lequel $X_1$ est un atome de chlore, $R_1$ un atome d'hydrogène et $R_2$ un groupe $=N—O—CH_2COOH$.

8. Composé selon la revendication 1 dans lequel $X_1$ est un atome de chlore, $R_1$ un atome d'hydrogène et $R_2$ un groupe

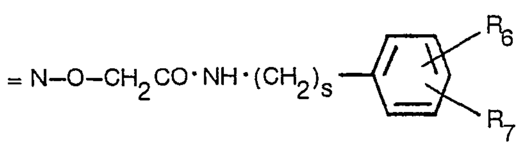

9. Composé selon la revendication 4, 6 ou 8 dans lequel $R_6$ est $^3H$ ou $^{127}I$.

10. Composé selon la revendication 9, dans lequel $R_6$ est $^{127}I$, $R_7$ un hydroxyle à la position 4 et s le nombre 2.

11. Composé selon la revendication 1 dans lequel $X_1$ est un atome de chlore, $R_2$ un atome d'oxygène et $R_1$ représente $^3H$ ou un radioisotope de l'iode.

12. Composé selon la revendication 11 dans lequel $R_1$ occupe la position 2.

13. Composé selon la revendication 1 dans lequel $R_2$ est un atome d'oxygène, $R_1$ un hydroxyle à la position 4 et $X_1$ représente $^3H$ ou un radioistope de l'iode.

14. Composé selon l'une quelconque des revendications 1 à 13 dans lequel le radioisotope de l'iode est l'iode 125.

15. Un procédé de préparation d'un composé de formule 1 selon la revendication 1, dans lequel $X_1$ est un atome de chlore, $R_2$ un atome d'oxygène et $R_1$ un groupe $—(CH_2)_n—OCO(CH_2)_pCOOH$, procédé selon lequel on fait réagir avec un anhydride de formule

$$OCO(CH_2)_pCO$$

un composé de formule 1 dans lequel $X_1$ est un atome de chlore, $R_2$ un atome d'oxygène et $R_1$ un groupe $—(CH_2)_n—OH$.

16. Un procédé se préparation d'un composé de formule 1 selon la revendication 1 dans lequel $X_1$ est un atome de chlore, $R_2$ un atome d'oxygène et $R_1$ un groupe $—O(CH_2)_nCOOH$, procédé selon lequel

on soumet à une hydrolyse en milieu basique un composé de formule 1 dans lequel $X_1$ est un atome de chlore, $R_2$ un atome d'oxygène et $R_1$ un gruope $—O(CH_2)_pCOOR_{11}$.

17. Un procédé de préparation d'un composé de formule 1 selon la revendication 1 dans lequel $X_1$ est un atome de chlore, $R_1$ un atome d'hydrogène et $R_2$ un groupe $=N·O(CH_2)_qCOOH$, procédé selon lequel on fait réagir la bupropion avec un hémichlorhydrate de carboxyalcoxylamine ayant de 1 à 3 atomes de carbone dans la partie alcoxy.

18. Un procédé de préparation d'un composé de formule 1 selon la revendication 1 dans lequel $X_1$ est un atome de chlore, $R_2$un atome d'oxygène et $R_1$ un groupe $—(CH_2)_n—O·CO(CH_2)_p—COZ$ ou $—O—(CH_2)_pCOZ$, Z représentant un groupe

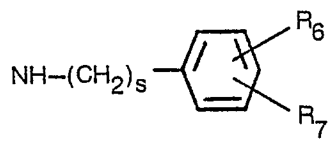

procédé selon lequel on fait réagir un composé de formule 1 dans lequel $X_1$ est un atome de chlore, $R_2$ un atome d'oxygène et $R_1$ un groupe $—(CH_2)_n—OCO(CH_2)_pCOOH$ ou $—O—(CH_2)_pCOOH$ avec un halogénoformiate d'alkyle ayant de 1 à 4 atomes de carbone dans la partie alkylique, en présence d'une trialkylamine, on fait ensuite réagir le produit ainsi formé avec une amine de formule

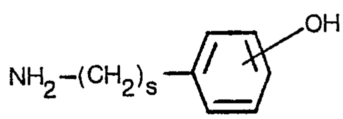

puis on fixe le nuclide radioactif sur le cycle de l'amine.

19. Procédé selon la revendication 18 dans lequel l'halogénoformiate est le chloroformiate d'isobutyle, la trialkylamine est la triéthylamine et l'amine est la tyramine.

20. Un procédé de préparation d'un composé de formule 1 selon la revendication 1 dans lequel $X_1$ est un atome de chlore, $R_1$ un atome d'hydrogène et $R_2$ un groupe

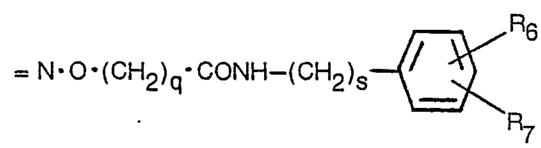

procédé selon lequel on fait réagir un composé de formule 1 dans lequel $X_1$ est un atome de chlore, $R_1$ un atome d'hydrogène et $R_2$ un groupe $–N.O.(CH_2)_qCOOH$ avec une amine de formule

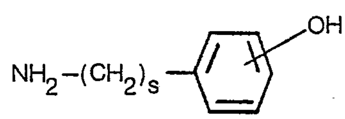

puis on fixe le nuclide radioactif sur le cycle de l'amine.

21. Procédé selon la revendication 20 selon lequel la réaction entre l'acide et l'amine est effectuée en présence de dicyclohexylcarbodiimide.

22. Procédé selon la revendication 20 ou 21 dans lequel l'amine est la tyramine.

23. Un immunogène comprenant un conjugé d'un support immunogène et d'un composé selon l'une quelconque des revendications 1 à 5 et 7 dans lequel $R_1$ est un groupe $—(CH_2)_n—O—(CO)_m—(CH_2)_pCOOH$ ou $—O—(CH_2)_pCOOH$, ou bien $R_2$ est un groupe $=N.O.(CH_2)_qCOOH$.

24. Un immunogène selon la revendication 23 dans lequel le support est de l'albumine de sérum de bovin.

25. Une méthode de prépartion d'un immunogène tel que défini à la revendication 23 ou 24 selon laquelle on fait réagir un support, immunogène avec un composé selon l'une quelconque des revendications 1 à 5 et 7, dans lequel $R_1$ est un groupe $—(CH_2)_n—O—(CO)_m·(CH_2)_pCOOH$ ou ou $—O—(CH_2)_pCOOH$ ou $R_2$ est un groupe $=N·O·(CH_2)_pCOOH$, en présence d'un agent de couplage.

26. Une méthode de production d'un anti-sérum contenant des anti-corps pour la bupropion et des métabolities actifs de cette substance, selon laquelle on apporte au système de réponse immunitaire d'un animal hôte un immunogène selon la revendication 23 ou 24 et on receuille du sang de l'animal le sérum contenant les anti-corps.

27. Méthode selon la revendiction 26 dans laquelle l'animal hôte est un lapin.

28. Une méthode pour déterminer la teneur en bupropion et en tous métabolities actifs de cette substance d'un liquide de l'organisme, méthode selon laquelle a) on mélange le liquide avec (1) un anti-sérum qui a été obtenu par une méthode selon la revendication 26 ou 27 et (2) un compétiteur de bupropion radiomarqué qui est un composé de formule 1 selon l'une quelconque des revendications 1, 2, 4, 6 et 8 à 14, comprenant un nuclide radioactif et pouvant entrer en compétition avec la bupropion pour se lier à l'anti-sérum; b) on mélange un échantillon de concentration connue en bupropion avec un anti-sérum (1) et un compétiteur radiomarqué (2); et c) on dose la quantité du compétiteur radiomarqué lié ou non à l'anti-sérum dans chacune des étapes a) et b) et on compare entre eux les résultats obtenus pour ces deux étapes.

29. Méthode selon la revendication 28 dans laquelle le compétiteur, l'anti-sérum et le liquide sont mélangés en présence d'une quantité suffisante d'un tampon pour donner un pH d'environ 6 à 9.

30. Méthode selon la revendication 28 ou 29 dans laquelle l'anti-sérum non lié et le liquide sont éliminés comme partie du dosage.

31. Méthode selon l'une quelconque des revendications 28 à 30 dans laquelle le liquide est du plasma sanguin ou du sérum sanguin.

32. Méthode selon l'une quelconque des revendications 28 à 31 dans laquelle on répète l'étape b) un nombre de fois suffisant en faisant varier la concentration connue en bupropion en vue d'obtenir les informations nécessaires à l'établissement d'une courbe étalonnée permettant d'inter-poler la quantité de bupropion et de tous métabolites actifs de cette substance pouvant se trouver dans le liquide.

33. Méthode selon l'une quelconque des revendications 28 à 32 dans laquelle on détermine la concentration en bupropion et métabolite actif d'après une courbe étalonnée qui représente le degré % d'inhibition de la liaison du compétiteur à l'anti-sérum par la bupropion.

34. Méthode selon l'une quelconque des revendications 28 à 33 dans laquelle la concentration du liquide est inférieur à 10% environ.

35. Méthode selon l'une quelconque des revendications 28 à 34 dans laquelle le mélange contient plus d'un microlitre du liquide.

36. Méthode selon l'une quelconque des revendications 28 à 35 dans laquelle le liquide est dialysé avant le mélange.

37. Méthode selon l'une quelconque des revendications 28 à 36 dans laquelle on élimine la bupropion non liée et le liquide avant de déterminer le degré % d'inhibition de la liaison.

38. Méthode selon l'une quelconque des revendications 28 à 37 dans laquelle, dans l'étape c), on a précipité l'antisérum en ajoutant au mélange de l'étanol refroidi par de la glace avant d'effecteur la mesure (dosage).

39. Méthode selon l'une quelconque des revendications 28 à 38 dans laquelle le compétiteur est 1'$\alpha$ - t - butylamino - 3 - chloropropiophénone - carboxyméthyloxime - $^{125}$I - tyramine amide; la $^{125}$I - $\alpha$ - t - butylamino - 3 - iodo - 4 - hydroxypropiophénone; le chlorhydrate d'$\alpha$ - t - butylamino - 3 - chloro - 6 - [$^3$H] - propio - phénone, ou le composé

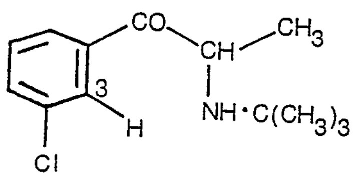

40. Méthode selon l'une quelconque des revendications 28 à 39 dans laquelle l'anti-sérum a été produit avec comme immunogène un conjugé d'$\alpha$ - t - butylamino - 3 - chloropropio - phénone - carboxyméthyloxime et d'albumine de sérum de bovin, et le compétiteur est l'$\alpha$ - t - butylamino - 3 - chloropropiophénone - carboxyméthyloxime - $^{125}$I - tyramine amide.

41. Une trousse d'essai comprenant un premier composant qui est un anti-sérum obtenu par une méthode selon la revendication 26 ou 27, et un second composant séparé dans la trousse qui est un compétiteur radiomarqué pour la bupropion, à savoir un composé de formule I tel que défini à l'une quelconque des revendications 1, 2, 4, 6 et 8 à 14, comprenant un nuclide radioactif.

42. Trousse selon la revendication 41 contenant un troisième composant mis séparément, qui est la bupropion.

43. Trousse selon la revendication 41 ou 42 dans laquelle l'anti-sérum a été produit avec comme immunogène un conjugué d'$\alpha$ - t - butylamino - 3 - chloropropiophénone - carboxyméthyloxime et d'albumine de sérum de bovin, et le compétiteur est l'$\alpha$ - t - butylamino - 3 - chloropropio-phénone - carboxyméthyloxime - $^{125}$I - tyramine amide.

Standard Curve of Example XI

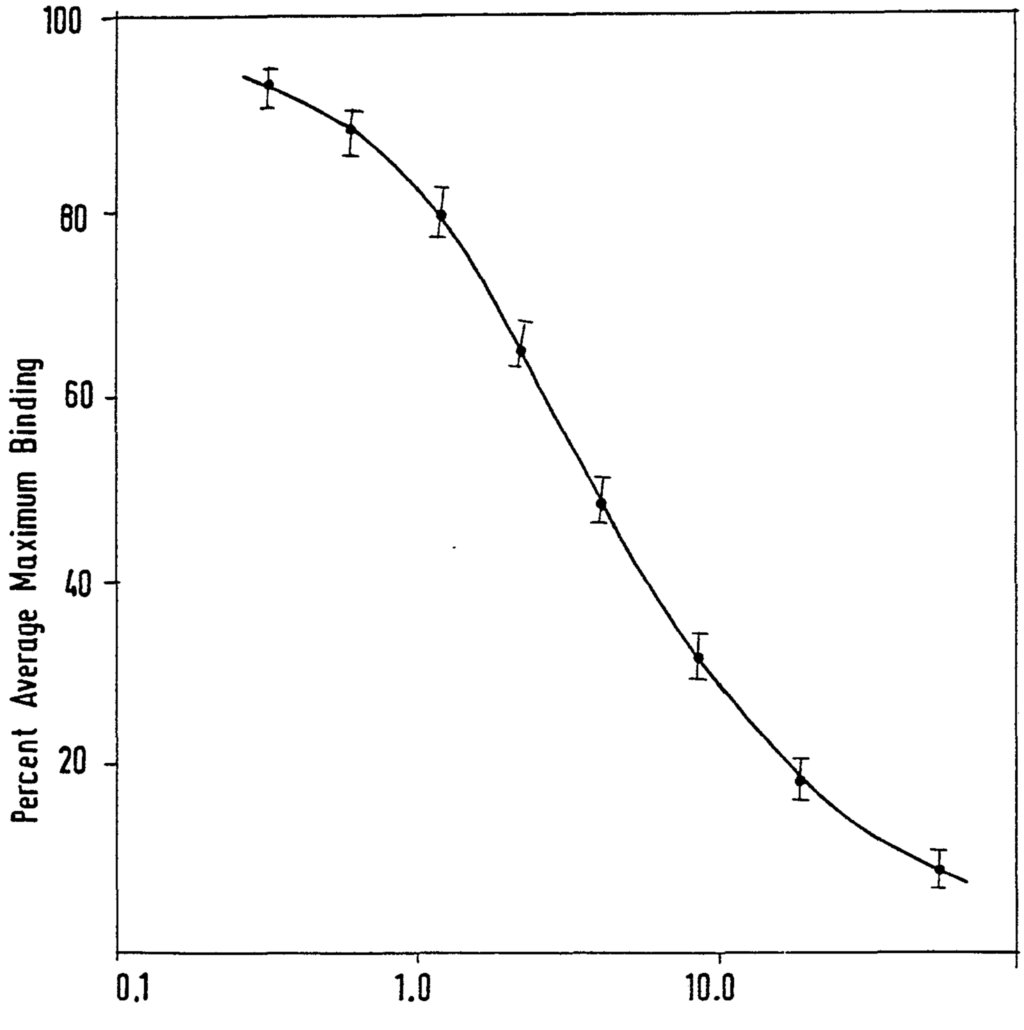


FIG. 1

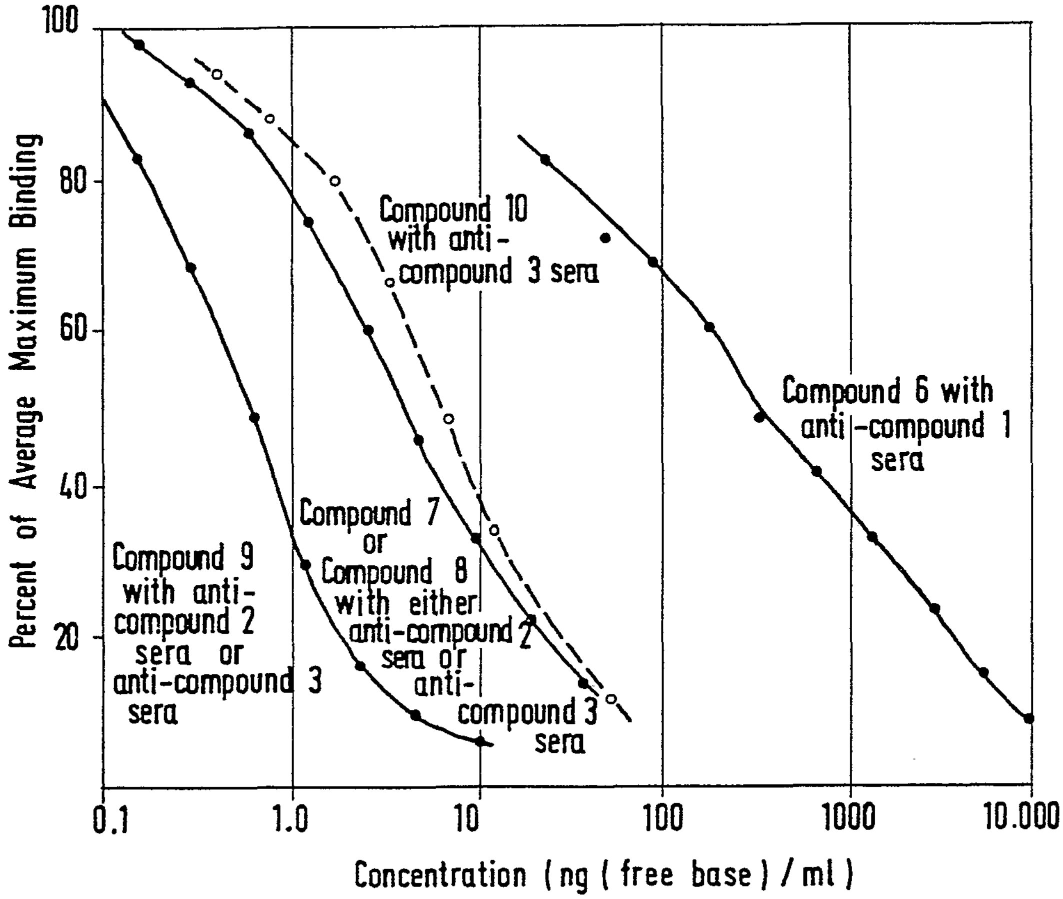
100
80
60
40
20
Percent of Average Maximum Binding
0.1
1.0
10
100
1000
10.000
Concentration (ng (free base) / ml)
Compound 10 with anti-compound 3 sera
Compound 6 with anti-compound 1 sera
Compound 7 or Compound 8 with either anti-compound 2 sera or anti-compound 3 sera
Compound 9 with anti-compound 2 sera or anti-compound 3 sera

FIG. 2